# EUROPÄISCHE PATENTANMELDUNG

(11) **EP 2 264 008 A1**
(43) Veröffentlichungstag der Anmeldung: **22.12.2010**
(21) Anmeldenummer: 09163137.4
(22) Anmeldetag: 18.06.2009
(51) Int. Cl.: C07D 213/61, C07D 405/12, A01N 43/40

(54) **Substituierte Enaminocarbonylverbindungen**

(71) Anmelder: Bayer CropScience AG, 40789 Monheim (DE)
(72) Erfinder: Die Erfindernennung liegt noch nicht vor
(74) Vertreter: Krieg, Robert Alexander

(57) **Zusammenfassung**

Die vorliegende Anmeldung betrifft subtituierte Enaminocarbonylverbindungen der Formel I 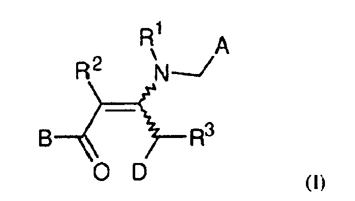 worin A, B, D, R¹ bis R³ die in der Beschreibung angegebenen Bedeutungen haben, Verfahren zu ihrer Herstellung und ihre Verwendung zur Bekämpfung von bierischen Schädlingen.

## Beschreibung

Die vorliegende Anmeldung betrifft substituierte Enaminocarbonylverbindungen, Verfahren zu Ihrer Herstellung und ihre Verwendung zur Bekämpfung von tierischen Schädlingen, vor allem von Arthropoden, insbesondere Insekten.

Substituierte Enaminocarbonylverbindungen wie beispielsweise γ-Allyloxy-β-enaminoester sind bekannt (vergleiche I. Pevet et al., Synlett 5, 663-666, 2003) und manche werden als Intermediate für die Synthese verschiedener Heterocyclen, wie beispielsweise 3,4,5-trifunktionalisierten Pyridinen, *N*-Methyl-dioxoindolen, Indol-3-carbonsäure Derivaten oder Pyrrolen, eingesetzt (vgl. γ-Methoxy-β-enamino-methylester: N. Nishiwaki et al., Synlett 9, 1437-1439, 2006; E. Comer, W. S. Murphy, ARKIVOC (Gainesville, FL, USA) 7, 286-296, 2003; M. Kinugawa et al., J. Chem. Soc., Perkin Trans. 1: Org. and Bio-Organic Chem. 21, 2677-2678, 1995; EP 565 050 A1; γ-Methoxy-β-enamino-methylester mit β-Vinylbromid Funktionalität: R. Grigg, V. Savic Chem. Commun. (Cambridge) 10, 873-874, 2000).

Moderne Planzenschutzmittel müssen vielen Anforderungen genügen, beispielsweise in Bezug auf Höhe, Dauer und Breite ihrer Wirkung und möglichen Verwendung. Es spielen Fragen der Toxizität eine Rolle sowie die Frage der Aufwandmenge sowie der Wirschaftlichkeit. Die Suche nach neuen Pflanzenschutzmitteln kann deshalb nie als abgeschlossen betrachtet werden und es besteht ständig Bedarf an neuen Verbindungen mit verbesserten Eigenschaften.

Aufgabe der vorliegenden Erfindung ist es, Verbindungen bereitzustellen, die die oben genannten Aspekte berücksichtigen.

Es wurde nun überraschenderweise gefunden, dass bestimmte substituierte Enaminocarbonylverbindungen sowie deren *N*-Oxide und Salze sich insbesondere zur Bekämpfung von tierischen Schädlingen eignen, und deshalb besonders gut im agrochemischen Bereich und im Bereich der Tiergesundheit einsetzbar sind.

Diese Enaminocarbonylverbindungen sind durch die Formel (I) 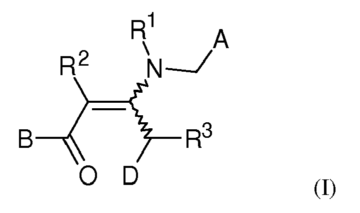 gekennzeichnet, worin
A für einen gegebenenfalls substituierten Heterocyclus steht;
B für einen Rest Z-R⁴ steht, worin
Z für Sauerstoff, Schwefel, oder gegebenenfalls substituierten Stickstoff steht, und
R⁴ für Wasserstoff, C₁-C₆-Alkyl, C₁-C₆-Alkoxy-C₁-C₆-alkyl, C₁-C₆-Alkylthio-C₁-C₆-alkyl, Halogen-C₁-C₆-alkyl, Cyan-C₁-C₆-alkyl, C₂-C₆-Alkenyl, C₂-C₆-Alkinyl, C₃-C₆-Cycloalkyl, C₄-C₁₀-Dicycloalkyl, Tri-C₁-C₆-alkylsilyl, Tri-C₁-C₆-alkylsilyl-C₁-C₆-alkyl, Hetaryl, Hetaryl-C₁-C₆-alkyl, gegebenenfalls substituiertes Aryl, Aryl-C₁-C₆-alkyl oder Aryl-C₁-C₆-alkoxy-C₁-C₆-alkyl steht;
D für Halogen, oder einem Rest T-R⁵ steht, worin
T für Sauerstoff, Schwefel oder gegebenenfalls substituierten Stickstoff steht, und
R⁵ für Wasserstoff, Formyl, C₁-C₆-Alkyl, C₁-C₆-Alkoxy-C₁-C₆-alkyl, Halogen-C₁-C₆-alkoxy-C₁-C₆-alkyl, C₁-C₆-Alkoxy-C₁-C₆-alkoxy-C₁-C₆-alkyl, C₁-C₆-Alkylthio-C₁-C₆-alkyl, C₂-C₆-Alkenyl, C₃-C₆-Cycloalkyl, C₂-C₆- Alkinyl, C₁-C₆-Alkoxycarbonyl, Aryl, Hetaryl, C₁-C₆-Alkylcarbonyl, Aryl-C₁-C₆-alkyl, Hetaryl-C₁-C₆-alkyl, Halogen-C₁-C₆-alkylcarbonyl, Arylcarbonyl, C₁-C₆-Alkylsulfonyl, Halogen-C₁-C₆-alkylsulfonyl, Aryl-C₁-C₆-alkyloxy-C₁-C₆-alkyl, Aryloxy-C₁-C₆-alkyl, Arylsulfonyl oder Tri-C₁-C₆-alkylsilyl steht;
R¹ für Wasserstoff, C₁-C₆-Alkyl, C₂-C₆-Alkenyl, C₁-C₆-Alkinyl, C₃-C₆-Cycloalkyl, C₃-C₆-Cycloalkyl-C₁-C₆-alkyl, Halogen-C₁-C₆-alkyl, Halogen-C₂-C₆-alkenyl, Halogen-C₃-C₆-cycloalkyl, Halogen-C₃-C₆-cycloalkyl-C₁-C₆-alkyl, C₁-C₆-Alkoxy oder Aryl-C₁-C₆-alkyl steht;
R² für Wasserstoff oder Halogen steht; und
R³ für Wasserstoff oder C₁-C₆-Alkyl steht.

Bevorzugte Enaminocarbonylverbindungen sind solche, in denen
A für einen Heterocyclus der folgenden Formel steht 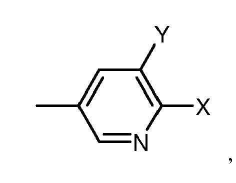 wobei
X für Halogen, vorzugsweise Fluor Chlor und Brom, C₁-C₆-Alkyl oder Halogen-C₁-C₆-alkyl, vorzugsweise CH₂F, CHF₂, CF₃, CH₂CF₃, CH₂CH₂F, CH₂CHF₂ steht und
Y für Halogen, vorzugsweise Fluor Chlor und Brom, C₁-C₆-Alkyl, Halogen-C₁-C₆-alkyl, vorzugsweise CH₂F, CHF₂, CF₃, CH₂CF₃, CH₂CH₂F, CH₂CHF₂, Halogen-C₁-C₆-alkoxy, vorzugsweise OCH₂F, OCHF₂, OCF₃, OCH₂CF₃, OCH₂CH₂F, OCH₂CHF₂, Azido oder Cyan steht; wobei bevorzugte Heterocyclen der vorgenannten Formel 5,6-Dibrom-pyrid-3-yl, 6-Brom-5-chlor-pyrid-3-yl, 6-Brom-5-fluor-pyrid-3-yl, 5-Chlor-6-iod-pyrid-3-yl, 5,6-Dichlor-pyrid-3-yl, 6-Chlor-5-fluor-pyrid-3-yl, 5-Brom-6-chlor-pyrid-3-yl, 6-Chlor-5-methyl-pyrid-3-yl, 6-Chlor-5-difluormethyl-pyrid-3-yl, 6-Chlor-1,4-pyridazin-3-yl, 2-Chlor-pyrimid-5-yl und 2-Chlor-1,3-thiazol-5-yl sind,
oder
A steht für eine der folgenden Gruppierungen Pyrid-2-yl; Pyrid-4-yl; oder Pyrid-3-yl, das gegebenenfalls in 6-Position substituiert ist durch Fluor, Chlor, Brom, Iod, Methyl, Difluormethyl, Trifluormethyl oder Trifluormethoxy; Pyridazin-3-yl, das gegebenenfalls in 6-Position substituiert ist durch Chlor oder Methyl; Pyrazin-3-yl; 2-Chlor-pyrazin-5-yl; 1,3-Thiazol-5-yl, welches gegebenenfalls in 2-Position substituiert ist durch Chlor oder Methyl; Tetrahydrofuryl; Pyrimidinyl; Pyrazolyl; Thiophenyl; Oxazolyl; Isoxazolyl; 1,2,4-Oxadiazolyl; Isothiazolyl; 1,2,4-Triazolyl; 1,2,5-Thiadiazolyl, das gegebenenfalls durch Fluor, Chlor, Brom, Cyano, Nitro, substituiert ist, oder mit jeweils gegebenenfalls mit Fluor und/oder Chlor substituiertem C₁-C₄-Alkyl C₁-C₃-Alkylthio, oder C₁-C₃-Alkylsulfonyl substituiert ist, bevorzugt für Pyrid-3-yl, welches in 6-Position durch Fluor, Chlor, Brom, Methyl oder Trifluormethyl oder Trifluormethoxy substituiert ist, d.h. 6-Fluor-pyrid-3-yl, 6-Chlor-pyrid-3-yl, 6-Brom-pyrid-3-yl und 6-Trifluormethyl-pyrid-3-yl, 6-Methyl-pyrid-3-yl, 6-Trifluormethoxypyrid-3-yl, oder für 2-Methyl-1,3-thiazol-5-yl, 2-Chlor-1,3-thiazol-5-yl, 2-Chlor-1,3-thiazol-5-yl, 2-Chlor-pyrazin-5-yl, 6-Chlor-1,4-pyridazin-3-yl, 6-Methyl-1,4-pyridazin-3-yl, ganz besonders bevorzugt für 6-Fluor-pyrid-3-yl, 6-Chlor-pyrid-3-yl, 6-Brompyrid-3-yl, 6-Chlor-1,4-pyridazin-3-yl oder 2-Chlor-1,3-thiazol-5-yl;
B für einen Rest Z-R⁴ steht, worin
Z für Sauerstoff oder Schwefel, bevorzugt für Sauerstoff steht, und
R⁴ für Wasserstoff, C₁-C₆-Alkyl, C₁-C₄-Alkoxy-C₁-C₆-alkyl, C₁-C₄-Alkylthio-C₁-C₆-alkyl, Halogen-C₁-C₆-alkyl, Cyan-C₁-C₄-alkyl, C₂-C₆-Alkenyl, C₂-C₆-Alkinyl, C₃-C₆-Cycloalkyl, Di-C₃-C₆-cycloalkyl-C₁-C₂-alkyl, Tri-C₁-C₆-alkylsilyl, Tri-C₁-C₆-alkylsilyl-C₁-C₂-alkyl, Hetaryl, Hetaryl-C₁-C₂-alkyl, Aryl-C₁-C₂-alkyl, Aryl-C₁-C₂-alkyloxy-C₁-C₃-alkyl, Phenyl oder einem mit Nitro, Halogen, C₁-C₄-Alkoxy, C₁-C₄-Alkylthio, C₁-C₄-Alkylsulfinyl oder C₁-C₄-Alkylsulfonyl substituiertem Phenyl, bevorzugt für Wasserstoff, Methyl, Ethyl, *n*-Propyl, *iso*-Propyl, *n*-Butyl, *iso*-Butyl, *sec*-Butyl, *tert-Butyl,* Methoxyethyl, Methylthiomethyl, 2-Methylthioethyl, 2,2,2-Trichlorethyl, 2-Chlor-ethyl, 2-Brom-ethyl, 2-Iod-ethyl, 2-Cyan-ethyl, Allyl, Methallyl, 3-Buten-1-yl, Propargyl, Cyclopentyl, Cyclohexyl, Dicyclopropylmethyl, Trimethylsilyl, Di-tert-butylmethylsilyl, *iso*-Propyldimethylsilyl, Trimethylsilylmethyl, 2-(2'-Pyridyl)ethyl, 4-Picolyl, Benzyl, 4-Methoxy-benzyl, 2,4-Dimethoxy-benzyl, 4-Brom-benzyl, 4-Methylsulfinyl-benzyl, 4-Nitro-benzyl, Benzyloxymethyl, 4-Methylthio-phenyl, 4-Nitro-phenyl oder 2,3,4,5,6-Pentafluor-phenyl, besonders bevorzugt für Wasserstoff, Methyl, Ethyl oder *iso*-Propyl steht;
D für Halogen, bevorzugt Chlor oder Brom oder einem Rest T-R⁵ steht, worin
T für Sauerstoff, Schwefel oder gegebenenfalls substituierten Stickstoff bevorzugt Sauerstoff, steht, und
R⁵ für Wasserstoff, Formyl, C₁-C₆-Alkyl, C₁-C₄-Alkoxy-C₁-C₆-alkyl, Halogen-C₁-C₄-alkoxy-C₁-C₆-alkyl, C₁-C₄-Alkoxy-C₁-C₄-alkoxy-C₁-C₆-alkyl, C₁-C₄-Alkylthio-C₁-C₆-alkyl, C₂-C₆-Alkenyl, C₃-C₆-Cycloalkyl, C₂-C₆-Alkinyl, C₁-C₆-Alkoxycarbonyl, Aryl, Tetrahydropyran-2-yl, 3-Brom-tetrahydropyran-2-yl, Tetrahydrothiopyran-2-yl, 1,4-Dioxan-2-yl, Tetrahydrofuranyl, C₁-C₆-Alkylcarbonyl, Aryl-C₁-C₂-alkyl, Hetaryl-C₁-C₂-alkyl, Halogen-C₁-C₆-alkylcarbonyl, Arylcarbonyl, C₁-C₆-Alkylsulfonyl, Halogen-C₁-C₆-alkylsulfonyl, Aryl-C₁-C₄-alkyloxy-C₁-C₄-alkyl, Aryloxy-C₁-C₄-alkyl, Arylsulfonyl oder Tri-C₁-C₆-alkylsilyl, bevorzugt für Wasserstoff, Formyl, Methyl, Ethyl, *n*-Propyl, *iso*-Propyl, *n*-Butyl, *iso*-Butyl, *sec*-Butyl, *tert*-Butyl, Allyl, Methoxymethyl, 1-Ethoxy-ethyl, *tert*-Butoxymethyl, Methylthiomethyl, Methoxyethoxymethyl, 1-(2-Chlorethoxy)ethyl, 1-Methyl-1-methoxyethyl, Cyclopropyl, Cyclobutyl, Propargyl, Methoxycarbonyl, Ethoxycarbonyl, Cyclopropyloxycarbonyl, Phenyl, Tetrahydropyran-2-yl, Methylcarbonyl, Ethylcarbonyl, *n*-Propylcarbonyl, *iso*-Propylcarbonyl, *tert*-Butylcarbonyl, Trifluormethylcarbonyl, 4-Nitro-phenylcarbonyl, 2,4-Dinitro-phenyl, 4-Nitrophenyl, Benzyl, 4-Methoxy-benzyl, 2,4-Dimethoxy-benzyl, 3,4-Dimethoxy-benzyl, 4-Nitrobenzyl, 2,6-Dichlorbenzyl, 4-Methoxy-benzyloxymethyl, 4-Nitro-benzyloxymethyl, 2-Picolyl, 4-Picolyl, Methylsulfonyl, Ethylsulfonyl, 4-Methoxy-phenyl, Trifluormethylsulfonyl, *para*-Toluolsulfonyl, Trimethylsilyl, Triethylsilyl, Triisopropylsilyl, Dimethylisopropylsilyl oder *tert*-Butyldimethylsilyl, besonders bevorzugt für Methylcarbonyl, Ethylcarbonyl, *tert*-Butylcarbonyl oder Methoxycarbonyl steht;
R¹ für Wasserstoff, C₁-C₆-Alkyl, C₂-C₆-Alkenyl, C₂-C₆-Alkinyl, C₃-C₆-Cycloalkyl, C₃-C₆-Cycloalkyl-C₁-C₄-alkyl, C₁-C₆-Alkoxy, Aryl-C₁-C₄-alkyl oder durch Fluor substituiertes C₁-C₆-Alkyl, C₂-C₆-Alkenyl, C₃-C₆-Cycloalkyl oder C₃-C₆-Cycloalkyl-C₁-C₄-alkyl, bevorzugt für Methyl, Ethyl, Allyl, Propargyl, 2-Fluor-ethyl, 2,2-Difluor-ethyl, Cyclopropyl, 2-Fluor-cyclopropyl oder Methoxy besonders bevorzugt für Methyl, Cyclopropyl oder 2,2-Difluor-ethyl steht;
R² für Wasserstoff, Fluor oder Chlor steht; und
R³ für Wasserstoff oder C₁-C₆-Alkyl steht.

Die Erfindung umfasst ferner ein Verfahren zur Herstellung der erfindungsgemäßen Verbindungen umfassend die folgenden Reaktionsschritte
(a) Umsetzung einer Verbindung der Formel (II) 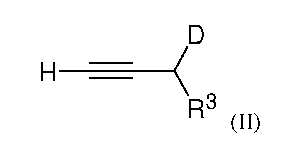 mit einer Verbindung der Formel (III) 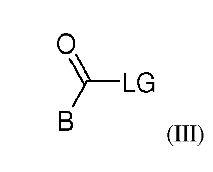 worin
   B, D und R³ die hier genannten Bedeutungen haben und LG für eine geeignete Abgangsgruppe (d.h. Leaving group) steht, insbesondere für Halogen-C₁-C₈-alkoxy, C₁-C₈-Alkanoyloxy, Mercapto, C₁-C₈-Alkylthio, Halogen-C₁-C₈-alkylthio, Halogen steht, vorzugsweise für ein C₁-C₈-Alkoxy wie Methoxy, Ethoxy, oder iso-Propoxy,
   vorzugsweise in Gegenwart eines basischen Hilfsmittels und eines Verdünnungsmittels zu einer Verbindung der Formel (IV); und
(b) Umsetzen der Verbindungen der Formel (IV) 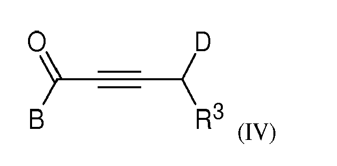 mit einer Verbindungen der Formel (V)

   HN(R¹)-CH₂-A (V)

   worin die B, D, A und R¹, und R³ die hier genannte Bedeutungen haben, vorzugsweise in Gegenwart eines Verdünnungsmittels.

Die Erfindung umfasst weiterhin ein Mittel zur Bekämpfung tierischer Schädlinge, vorzugsweise Arthropoden, insbesondere Insekten das mindestens eine erfindungsgemäße Verbindung in einer insektizid wirksamen Menge beinhaltet.

Die Erfindung umfasst ferner die Verwendung einer erfindungsgemäßen Verbindung zur Bekämpfung tierischer Schädlinge im agrochemischen Bereich und/oder im Bereich der Tiergesundheit sowie ein Verfahren zum Bekämpfen tierischer Schädlinge, dadurch gekennzeichnet, dass man eine erfindungsgemäße Verbindung oder ein erfindungsgemäßes Mittel auf die Schädlinge und/oder ihren Lebensraum einwirken lässt.

Die erfindungsgemäßen Verbindungen können in Abhängigkeit von der Art der Substituenten als geometrische und/oder als optisch aktive Isomere oder entsprechende Isomerengemische in unterschiedlicher Zusammensetzung vorliegen. Diese Stereoisomere sind beispielsweise Enantiomere, Diastereomere, Atropisomere oder geometrische Isomere. Die Erfindung umfasst somit reine Stereoisomere als auch beliebige Gemische dieser Isomere.

In einer hervorgehobenen Gruppe von Verbindungen der Formel (I) stehen R³ für Wasserstoff, B für *iso*-Propoxy und A für 6-Chlor-pyrid-3-yl.

In einer weiteren hervorgehobenen Gruppe von Verbindungen der Formel (I) stehen R³ für Wasserstoff, B für *iso*-Propoxy und A für 6-Brom-pyrid-3-yl.

In einer weiteren hervorgehobenen Gruppe von Verbindungen der Formel (I) stehen R³ für Wasserstoff, B für *iso*-Propoxy und A für 6-Fluor-pyrid-3-yl.

In einer weiteren hervorgehobenen Gruppe von Verbindungen der Formel (I) stehen R³ für Wasserstoff, B für *iso*-Propoxy und A für 6-Trifluormethyl-pyrid-3-yl.

In einer weiteren hervorgehobenen Gruppe von Verbindungen der Formel (I) stehen R³ für Wasserstoff, B für *iso*-Propoxy und A für 2-Chlor-1,3-thiazol-5-yl.

In einer weiteren hervorgehobenen Gruppe von Verbindungen der Formel (I) stehen R³ für Wasserstoff, B für *iso*-Propoxy und A für 5,6-Dibrom-pyrid-3-yl.

In einer weiteren hervorgehobenen Gruppe von Verbindungen der Formel (I) stehen R³ für Wasserstoff, B für *iso*-Propoxy und A für 6-Brom-5-chlor-pyrid-3-yl.

In einer weiteren hervorgehobenen Gruppe von Verbindungen der Formel (I) stehen R³ für Wasserstoff, B für *iso*-Propoxy und A für 6-Brom-5-fluor-pyrid-3-yl.

In einer weiteren hervorgehobenen Gruppe von Verbindungen der Formel (I) stehen R³ für Wasserstoff, B für *iso*-Propoxy und A für 5-Chlor-6-iod-pyrid-3-yl.

In einer weiteren hervorgehobenen Gruppe von Verbindungen der Formel (I) stehen R³ für Wasserstoff, B für *iso*-Propoxy und A für 5,6-Dichlor-pyrid-3-yl.

In einer weiteren hervorgehobenen Gruppe von Verbindungen der Formel (I) stehen R³ für Wasserstoff, B für *iso*-Propoxy und A für 6-Chlor-5-fluor-pyrid-3-yl.

In einer weiteren hervorgehobenen Gruppe von Verbindungen der Formel (I) stehen R³ für Wasserstoff, B für *iso*-Propoxy und A für 5-Brom-6-chlor-pyrid-3-yl.

In einer weiteren hervorgehobenen Gruppe von Verbindungen der Formel (I) stehen R³ für Wasserstoff, B für *iso*-Propoxy und A für 6-Chlor-5-methyl-pyrid-3-yl.

In einer weiteren hervorgehobenen Gruppe von Verbindungen der Formel (I) stehen R³ für Wasserstoff, B für *iso*-Propoxy und A für 6-Chlor-5-difluormethyl-pyrid-3-yl.

In einer weiteren hervorgehobenen Gruppe von Verbindungen der Formel (I) stehen R² und R³ für Wasserstoff, B für *iso*-Propoxy und A für 6-Chlor-pyrid-3-yl.

In einer weiteren hervorgehobenen Gruppe von Verbindungen der Formel (I) stehen R² und R³ für Wasserstoff, B für *iso*-Propoxy und A für 6-Brom-pyrid-3-yl.

In einer weiteren hervorgehobenen Gruppe von Verbindungen der Formel (I) stehen R² und R³ für Wasserstoff, B für *iso*-Propoxy und A für 6-Fluor-pyrid-3-yl.

In einer weiteren hervorgehobenen Gruppe von Verbindungen der Formel (I) stehen R² und R³ für Wasserstoff, B für *iso*-Propoxy und A für 6-Trifluormethyl-pyrid-3-yl.

In einer weiteren hervorgehobenen Gruppe von Verbindungen der Formel (I) stehen R² und R³ für Wasserstoff, B für *iso*-Propoxy und A für 2-Chlor-1,3-thiazol-5-yl.

In einer weiteren hervorgehobenen Gruppe von Verbindungen der Formel (I) stehen R² und R³ für Wasserstoff, B für *iso*-Propoxy und A für 5,6-Dibrom-pyrid-3-yl.

In einer weiteren hervorgehobenen Gruppe von Verbindungen der Formel (I) stehen R² und R³ für Wasserstoff, B für *iso*-Propoxy und A für 6-Brom-5-chlor-pyrid-3-yl.

In einer weiteren hervorgehobenen Gruppe von Verbindungen der Formel (I) stehen R² und R³ für Wasserstoff, B für *iso*-Propoxy und A für 6-Brom-5-fluor-pyrid-3-yl.

In einer weiteren hervorgehobenen Gruppe von Verbindungen der Formel (I) stehen R² und R³ für Wasserstoff, B für *iso*-Propoxy und A für 5-Chlor-6-iod-pyrid-3-yl.

In einer weiteren hervorgehobenen Gruppe von Verbindungen der Formel (I) stehen R² und R³ für Wasserstoff, B für *iso*-Propoxy und A für 5,6-Dichlor-pyrid-3-yl.

In einer weiteren hervorgehobenen Gruppe von Verbindungen der Formel (I) stehen R² und R³ für Wasserstoff, B für *iso*-Propoxy und A für 6-Chlor-5-fluor-pyrid-3-yl.

In einer weiteren hervorgehobenen Gruppe von Verbindungen der Formel (I) stehen R² und R³ für Wasserstoff, B für *iso*-Propoxy und A für 5-Brom-6-chlor-pyrid-3-yl.

In einer weiteren hervorgehobenen Gruppe von Verbindungen der Formel (I) stehen R² und R³ für Wasserstoff, B für *iso*-Propoxy und A für 6-Chlor-5-methyl-pyrid-3-yl.

In einer weiteren hervorgehobenen Gruppe von Verbindungen der Formel (I) stehen R² und R³ für Wasserstoff, B für *iso*-Propoxy und A für 6-Chlor-5-difluormethyl-pyrid-3-yl.

In einer weiteren hervorgehobenen Gruppe von Verbindungen der Formel (I) stehen R² und R³ für Wasserstoff, B für Methoxy und A für 6-Chlor-pyrid-3-yl.

In einer weiteren hervorgehobenen Gruppe von Verbindungen der Formel (I) stehen R² und R³ für Wasserstoff, B für Methoxy und A für 6-Brom-pyrid-3-yl.

In einer weiteren hervorgehobenen Gruppe von Verbindungen der Formel (I) stehen R² und R³ für Wasserstoff, B für Methoxy und A für 6-Fluor-pyrid-3-yl.

In einer weiteren hervorgehobenen Gruppe von Verbindungen der Formel (I) stehen R² und R³ für Wasserstoff, B für Methoxy und A für 6-Trifluormethyl-pyrid-3-yl.

In einer weiteren hervorgehobenen Gruppe von Verbindungen der Formel (I) stehen R² und R³ für Wasserstoff, B für Methoxy und A für 2-Chlor-1,3-thiazol-5-yl.

In einer weiteren hervorgehobenen Gruppe von Verbindungen der Formel (I) stehen R² und R³ für Wasserstoff, B für Methoxy und A für 5,6-Dibrom-pyrid-3-yl.

In einer weiteren hervorgehobenen Gruppe von Verbindungen der Formel (I) stehen R² und R³ für Wasserstoff, B für Methoxy und A für 6-Brom-5-chlor-pyrid-3-yl.

In einer weiteren hervorgehobenen Gruppe von Verbindungen der Formel (I) stehen R² und R³ für Wasserstoff, B für Methoxy und A für 6-Brom-5-fluor-pyrid-3-yl.

In einer weiteren hervorgehobenen Gruppe von Verbindungen der Formel (I) stehen R² und R³ für Wasserstoff, B für Methoxy und A für 5-Chlor-6-iod-pyrid-3-yl.

In einer weiteren hervorgehobenen Gruppe von Verbindungen der Formel (I) stehen R² und R³ für Wasserstoff, B für Methoxy und A für 5,6-Dichlor-pyrid-3-yl.

In einer weiteren hervorgehobenen Gruppe von Verbindungen der Formel (I) stehen R² und R³ für Wasserstoff, B für Methoxy und A für 6-Chlor-5-fluor-pyrid-3-yl.

In einer weiteren hervorgehobenen Gruppe von Verbindungen der Formel (I) stehen R² und R³ für Wasserstoff, B für Methoxy und A für 5-Brom-6-chlor-pyrid-3-yl.

In einer weiteren hervorgehobenen Gruppe von Verbindungen der Formel (I) stehen R² und R³ für Wasserstoff, B für Methoxy und A für 6-Chlor-5-methyl-pyrid-3-yl.

In einer weiteren hervorgehobenen Gruppe von Verbindungen der Formel (I) stehen R² und R³ für Wasserstoff, B für Methoxy und A für 6-Chlor-5-difluormethyl-pyrid-3-yl.

In einer weiteren hervorgehobenen Gruppe von Verbindungen der Formel (I) stehen R¹ für Difluormethyl, R² und R³ für Wasserstoff und B für *iso*-Propoxy.

In einer weiteren hervorgehobenen Gruppe von Verbindungen der Formel (I) stehen R¹ für 2-Fluorethyl, R² und R³ für Wasserstoff und B für *iso*-Propoxy.

In einer weiteren hervorgehobenen Gruppe von Verbindungen der Formel (I) stehen R¹ für 2,2-Difluorethyl, R² und R³ für Wasserstoff und B für *iso*-Propoxy.

In einer weiteren hervorgehobenen Gruppe von Verbindungen der Formel (I) stehen R¹ für Methyl, R² und R³ für Wasserstoff und B für *iso*-Propoxy.

In einer weiteren hervorgehobenen Gruppe von Verbindungen der Formel (I) stehen R¹ für Cyclopropyl, R² und R³ für Wasserstoff und B für *iso*-Propoxy.

In einer weiteren hervorgehobenen Gruppe von Verbindungen der Formel (I) stehen R¹ für 2-Fluor-cyclopropyl, R² und R³ für Wasserstoff und B für *iso*-Propoxy.

In einer weiteren hervorgehobenen Gruppe von Verbindungen der Formel (I) stehen R¹ für Methoxy, R² und R³ für Wasserstoff und B für *iso*-Propoxy.

In einer weiteren hervorgehobenen Gruppe von Verbindungen der Formel (I) stehen R¹ für Difluormethyl, R² und R³ für Wasserstoff und B für Methoxy.

In einer weiteren hervorgehobenen Gruppe von Verbindungen der Formel (I) stehen R¹ für 2-Fluorethyl, R² und R³ für Wasserstoff und B für Methoxy.

In einer weiteren hervorgehobenen Gruppe von Verbindungen der Formel (I) stehen R¹ für 2,2-Difluorethyl, R² und R³ für Wasserstoff und B für Methoxy.

In einer weiteren hervorgehobenen Gruppe von Verbindungen der Formel (I) stehen R¹ für Methyl, R² und R³ für Wasserstoff und B für Methoxy.

In einer weiteren hervorgehobenen Gruppe von Verbindungen der Formel (I) stehen R¹ für Cyclopropyl, R² und R³ für Wasserstoff und B für Methoxy.

In einer weiteren hervorgehobenen Gruppe von Verbindungen der Formel (I) stehen R¹ für 2-Fluor-cyclopropyl, R² und R³ für Wasserstoff und B für Methoxy.

In einer weiteren hervorgehobenen Gruppe von Verbindungen der Formel (I) stehen R¹ für Methoxy, R² und R³ für Wasserstoff und B für Methoxy.

In einer weiteren hervorgehobenen Gruppe von Verbindungen der Formel (I) stehen R¹ für Methyl, R² und R³ für Wasserstoff und B für *iso*-Propoxy.

In einer weiteren hervorgehobenen Gruppe von Verbindungen der Formel (I) stehen R¹ für Cyclopropyl, R² und R³ für Wasserstoff und B für *iso*-Propoxy.

In einer weiteren hervorgehobenen Gruppe von Verbindungen der Formel (I) stehen R¹ für Ethyl, R² und R³ für Wasserstoff und B für *iso*-Propoxy.

In einer weiteren hervorgehobenen Gruppe von Verbindungen der Formel (I) stehen R¹ für Methoxy, R² und R³ für Wasserstoff und B für *iso*-Propoxy.

In einer weiteren hervorgehobenen Gruppe von Verbindungen der Formel (I) stehen R¹ für 2-Fluor-cyclopropyl, R² und R³ für Wasserstoff und B für *iso*-Propoxy.

In einer weiteren hervorgehobenen Gruppe von Verbindungen der Formel (I) stehen R¹ für Methyl, R² und R³ für Wasserstoff und B für Methoxy.

In einer weiteren hervorgehobenen Gruppe von Verbindungen der Formel (I) stehen R¹ für Cyclopropyl, R² und R³ für Wasserstoff und B für Methoxy.

In einer weiteren hervorgehobenen Gruppe von Verbindungen der Formel (I) stehen R¹ für Ethyl, R² und R³ für Wasserstoff und B für Methoxy.

In einer weiteren hervorgehobenen Gruppe von Verbindungen der Formel (I) stehen R¹ für Difluormethyl, R² und R³ für Wasserstoff und D für Methylcarbonyl-oxy.

In einer weiteren hervorgehobenen Gruppe von Verbindungen der Formel (I) stehen R¹ für 2-Fluorethyl, R² und R³ für Wasserstoff und D für Methylcarbonyl-oxy.

In einer weiteren hervorgehobenen Gruppe von Verbindungen der Formel (I) stehen R¹ für 2,2-Difluorethyl, R² und R³ für Wasserstoff und D für Methylcarbonyl-oxy.

In einer weiteren hervorgehobenen Gruppe von Verbindungen der Formel (I) stehen R¹ für Difluormethyl, R² und R³ für Wasserstoff und D für Hydroxy.

In einer weiteren hervorgehobenen Gruppe von Verbindungen der Formel (I) stehen R¹ für 2-Fluorethyl, R² und R³ für Wasserstoff und D für Hydroxy.

In einer weiteren hervorgehobenen Gruppe von Verbindungen der Formel (I) stehen R¹ für 2,2-Difluorethyl, R² und R³ für Wasserstoff und D für Hydroxy.

In einer weiteren hervorgehobenen Gruppe von Verbindungen der Formel (I) stehen R¹ für Methyl, R² und R³ für Wasserstoff und B für D für Methylcarbonyl-oxy.

In einer weiteren hervorgehobenen Gruppe von Verbindungen der Formel (I) stehen R¹ für Cyclopropyl, R² und R³ für Wasserstoff und D für Methylcarbonyl-oxy.

In einer weiteren hervorgehobenen Gruppe von Verbindungen der Formel (I) stehen R¹ für Ethyl, R² und R³ für Wasserstoff und D für Methylcarbonyl-oxy.

In einer weiteren hervorgehobenen Gruppe von Verbindungen der Formel (I) stehen R¹ für Methyl, R² und R³ für Wasserstoff und D für Hydroxy.

In einer weiteren hervorgehobenen Gruppe von Verbindungen der Formel (I) stehen R¹ für Cyclopropyl, R² und R³ für Wasserstoff und D für Hydroxy.

In einer weiteren hervorgehobenen Gruppe von Verbindungen der Formel (I) stehen R¹ für Ethyl, R² und R³ für Wasserstoff und D für Hydroxy.

Die oben aufgeführten allgemeinen oder als bevorzugt aufgeführten Restedefinitionen bzw. Erläuterungen gelten für die Endprodukte und für die Ausgangsprodukte und Zwischenprodukte entsprechend. Diese Restedefinitionen können untereinander, also auch zwischen den jeweiligen Vorzugsbereichen, beliebig kombiniert werden.

Erfindungsgemäß bevorzugt werden die (*E*)-Isomere der Formel (I).

Erfindungsgemäß geeignete Salze der erfindungsgemäßen Verbindungen, beispielsweise Salze mit Basen oder Säureadditionssalze, sind alle üblichen nicht toxischen Salze, vorzugsweise landwirtschaftlich und/oder physiologisch annehmbare Salze. Beispielsweise Salze mit Basen oder Säureadditionssalze. Bevorzugt werden Salze mit anorganischen Basen, wie beispielsweise Alkalimetallsalze (z.B. Natrium-, Kalium- oder Cäsiumsalze), Erdalkalimetallsalze (z.B. Calzium- oder Magnesiumsalze), Ammoniumsalze oder Salze mit organischen Basen, insbesondere mit organischen Aminen, wie beispielsweise Triethylammonium-, Dicyclohexylammonium-, *N,N'-*Dibenzylethylendiammonium-, Pyridinium-, Picolinium- oder Ethanolammoniumsalze, Salze mit anorganischen Säuren (z.B. Hydrochloride, Hydrobromide, Dihydrosulfate, Trihydrosulfate, oder Phosphate), Salze mit organischen Carbonsäuren oder organischen Sulfosäuren (z.b. Formiate, Acetate, Trifluoracetate, Maleate, Tartrate, Methansulfonate, Benzolsulfonate oder 4-Toluolsulfonate). Bekannterweise können tertiäre Amine, wie beispielsweise manche der erfindungsgemäßen Verbindungen, *N*-Oxide bilden, welche ebenfalls erfindungsgemäße Salze darstellen.

Im Folgenden werden Herstellungsverfahren für die erfindungsgemäßen Verbindungen beschrieben, ohne dass diese einschränkend zu verstehen sind. Weitere Verfahren zur Herstellung der erfindungsgemäßen Verbindungen können in analoger Weise hergeleitet werden.

Wie im Reaktionsschema 1 gezeigt, können erfindungsgemäße Verbindungen erhalten werden, indem man bei dem Verfahren zur Herstellung der erfindungsgemäßen Verbindungen im Reaktionsschritt (a) beispielsweise Prop-2-in-1-yl-acetat (als Verbindung (II)) und Chlorameisensäure-*iso*-propylester (als Verbindung (III)) einsetzt. Der so erhaltene 4-Acetoxybut-2-in-carbonsäure-*iso*-propylester (als Verbindung (IV)) wird dann im Reaktionsschritt (b) mit beispielsweise *N*-[6-Chlorpyridin-3-yl)methyl]-methan-1-amin (als Verbindung (V)) umgesetzt.

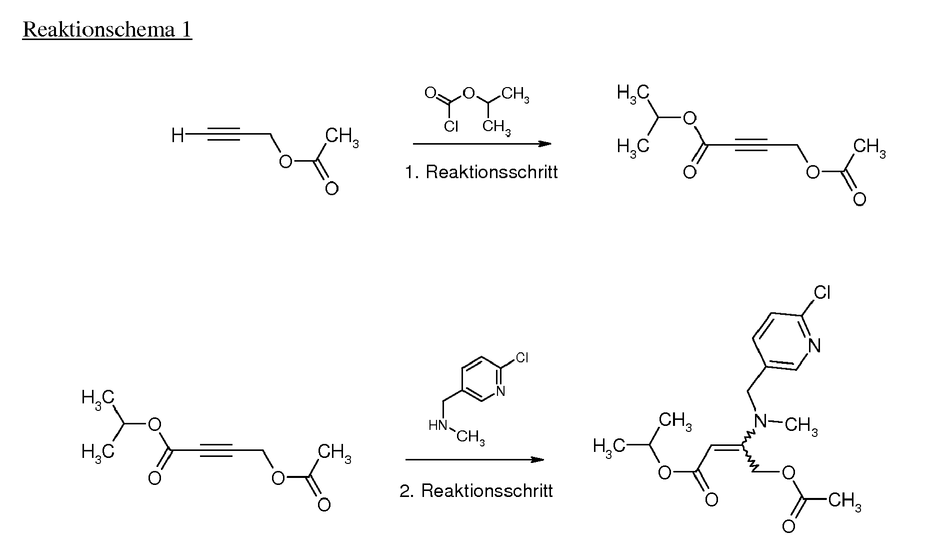

Verbindungen den Formel (II), worin die Reste D und R³ die oben definierten Bedeutungen haben, können teilweise kommerziell erhalten oder nach literaturbekannten Methoden synthetisiert werden (vgl. beispielsweise Verbindungen der Formel (II), worin D = O-CH(CH₃)₂, R³ = H: T. Takahashi, et al., Macromolecules 28, 866-869, 1995; D = O-CO-C(CH₃)₃, R³ = H: P. Prabhakar et al., Chem. Lett. 36, 732-733, 2007; D = O-CO-CH₃, R³ = CH₃: K Hiroi, F. Kato Tetrahedron 57, 1543-1550, 2001; D = O-CO-CH₃, R³ = C₂H₅: J. Kunes et al., Coll. Czech. Chem. Commun. 66, 1809-1830, 2001; C. Raminelli et al., Tetrahedron: Asymmetry 15, 3117-3122, 2004 [als *(S)*-Enantiomer]).

Verbindungen der Formel (III), worin der Rest B die oben definierte Bedeutung hat und LG beispielsweise für Halogen-C₁-C₈-alkoxy, C₁-C₈-Alkanoyloxy, Mercapto, C₁-C₈-Alkylthio, Halogen-C₁-C₈-alkylthio, Halogen steht, insbesondere für C₁-C₈-Alkoxy (z.B. Methoxy) steht, können zum Teil kommerziell (z. B. Chlorameisensäureester, Säurechloride) oder nach literaturbekannten Methoden erhalten werden.

Verbindungen der Formel (IV), worin die Reste B, D und R³ die oben definierten Bedeutungen haben, können zum Teil kommerziell oder nach literaturbekannten Methoden erhalten werden (vgl. beispielsweise Verbindungen der Formel (IV), worin B = OCH₂C₆H₅, D = OH, R³ = H: B. M. C. Clasby et al., Bioorg. Med. Chem. Lett. 13, 3647-3651, 2007; B = OCH(CH₃)₂, D = O-CO-O-CH₃, R³ = H: R. Tayama, R. Hashimoto Tetrahedron Lett. 48, 7950-7952, 2007; B = OCH₃, D = O-CO-O-CH₃, R³ = H: O. David et al., Heterocycles 62, 839-846, 2004; B = OCH₃, D = O-CH₂-C≡CH, R³ = H: Y. Yamamoto Tetrahedron Lett. 62, 4294-4305, 2006; B = OCH₃, D = O-C(CH₃)₃, R³ = H: A. Covarrubias-Zuniga et al., Synth. Commun. 28, 3461-3469, 1998; B = OCH₃, D = O-CO-CH₃, R³ = H: N. Maezaki et al., Tetrahedron: Asymmetry. 13, 1961-1964, 2002; B = OCH₃, D = Tetrahydro-2*H*-pyran-2-yl-oxy, R³ = H: R. Larock et al., J. Org. Chem.48, 2151-2158, 1983; B = OC₂H₅, D = O-CO-Aryl, R³ = H: Ch.-K. Jung et al., J. Am. Chem. Soc. 126, 4118-4119, 2004). Desweiteren ist die Herstellung und Verwendung bestimmter *O*-substituierter 4-Hydroxy-alkin-2-carbonsäureester als Mikrobiozide bekannt geworden (vgl. B = OC₂H₅, D = O-CO-n-C₄H₉, R³ = CH₃: DE-OS 4417752 A1).

Verbindungen der Formel (V), worin die Reste A und R¹ wie oben definiert sind, lassen sich auch aus Verbindungen der Formel (VI) nach literaturbekannten Methoden herstellen (vgl. *N*-[(6-Chlorpyridin-3-yl)methyl]-2,2-difluorethan-1-amin, *N*-[(6-Chlor-5-fluor-pyridin-3-yl)methyl]-2,2-difluorethan-1-amin: WO 2007/115646; WO 2008/ 009360; vgl. Schema 2 weiter unten).

Verbindungen der Formel (VI) sind zum Teil kommerziell erhältlich, bekannt oder lassen sich nach bekannten Methoden herstellen (z. B. 2-Chlor-5-chlormethyl-1,3-thiazol: DE 3 631 538 (1988), EP 446 913 (1991), EP 780 384 (1997), EP 775 700 (1997), EP 794 180 (1997), WO 9 710 226 (1997); 6-Chlor-3-chlormethyl-pyridin: DE 3 630 046 A1 (1988), EP 373 464 A2 (1990), EP 373 464 A2 (1990), EP 393 453 A2 (1990), EP 569 947 A1 (1993); 6-Chlor-3-brommethyl-pyridin: I. Cabanal-Duvillard et al., Heterocycl. Commun. 5, 257-262 (1999); 6-Brom-3-chlormethyl-pyridin, 6-Brom-3-hydroxymethyl-pyridin: US-Pat. 5 420 270 A (1995); 6-Fluor-3-chlormethyl-pyridin: J. A. Pesti et al., J. Org. Chem. 65, 7718-7722 (2000); 6-Methyl-3-chlormethyl-pyridin: EP 302389 A2, E. v der Eycken et al., J. Chem. Soc., Perkin Trans 2 5, 928-937 (2002); 6-Trifluormethyl-3-chlormethyl-pyridin: WO 2004/082616 A2; 2-Chlor-5-chlormethyl-pyrazin: JP 05239034 A2).

Allgemeine Wege zur Herstellung von Verbindungen der Formel (VI) sind im Reaktionsschema 2 wiedergegeben.

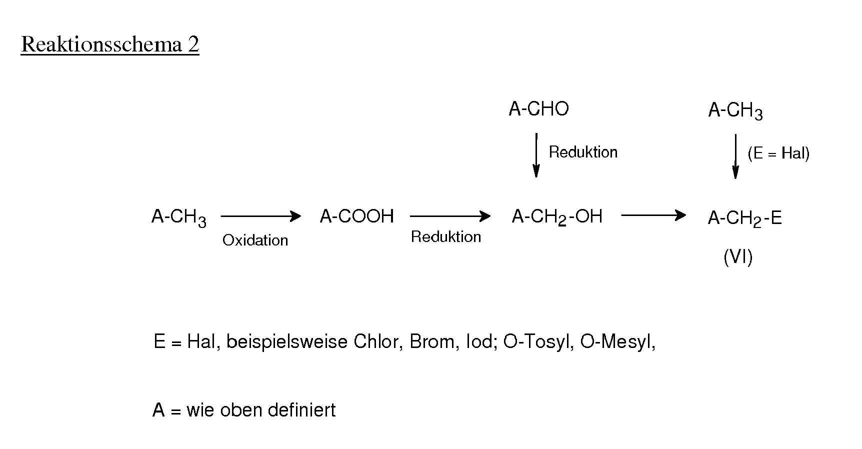

Die heterocyclischen Carbonsäure (A-COOH) kann nach literaturbekannten Methoden in die entsprechende heterocyclische Hydroxymethylverbindungen (A-CH₂-OH) überführt werden, die anschließend nach literaturbekannten Methoden zu aktivierten heterocyclischen Hydroxymethylverbindungen (A-CH₂-E, E = OTosyl, OMesyl) oder heterocyclischen Halogenmethylverbindungen (A-CH₂-E, E = Hal) umgesetzt werden. Letztere können auch aus entsprechenden Methylgruppe-haltigen Heterocyclen (A-CH₃) unter Verwendung von geeigneten literaturbekannten Halogenierungsmitteln erhalten werden.

Zur Darstellung von Verbindungen der Formel (V) ist es vorteilhaft, dass man beispielsweise Verbindungen der Formel (VI), in der die Reste A und E wie oben definiert sind, mit Verbindungen der Formel (VII), in der der Rest R¹ wie oben definiert ist in Gegenwart von Verdünnungsmitteln und in Gegenwart von basischen Reaktionshilfsmitteln umsetzt (vgl. *N*-Alkylierung, Reaktionsschema 3).

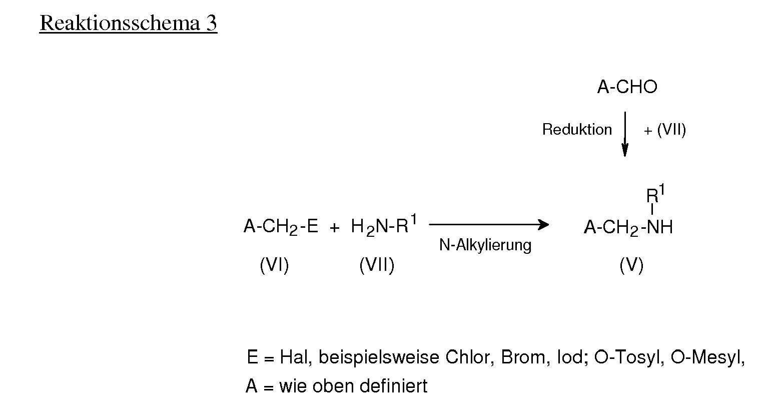

Die Aminoverbindungen der Formel (VII) können zum Teil kommerziell (vgl. z. B. 2-Fluorethylamin oder 2,2-Difluorethylamin) oder in an sich bekannter Weise nach der "Leuckart-Wallach-Reaktion (z. B. 2-Fluor-ethylamin: US-Pat. 4030994 (1977)) erhalten werden; Gleichfalls können Verbindungen der Formel (VII) in welcher R¹ für Alkyl steht erhalten werden (primäre Amine: vgl. z. B. Houben-Weyl, Methoden der Organischen Chemie, Bd. XI/1, 4. Aufl. 1957, Georg Thieme Verlag Stuttgart, S. 648; M. L. Moore in "The Leuckart Reaction" in: Organic Reactions, Bd. 5, 2. Aufl. 1952, New York, John Wiley & Sons, Inc. London) (vgl. z. B. auch 3-Fluor-n-propylamin: US 6252087 B1; 3,3-Difluor-prop-2-enylamin Hydrochlorid: WO 2001/007414 A1; 3,3-Dichlor-prop-2-enylamin: DE 2747814); 2-Chlor-2-fluor-cyclopropylamin, 2,2-Dichlor-cyclopropylamin: K. R. Gassen, B. Baasner, J. Fluorine Chem. 49, 127-139, 1990).

Alternativ können bestimmte Aminoverbindungen der Formel (VIIa), in welcher R¹ für CH₂-R' (R' = halogenhaltiger Rest; Halogen = Fluor oder Chlor) steht, auch durch Reduktion von halogenierten Carbonsäureamiden (VIII) in Gegenwart geeigneter Reduktionsmittel erhalten werden (Reaktionsschema 4).

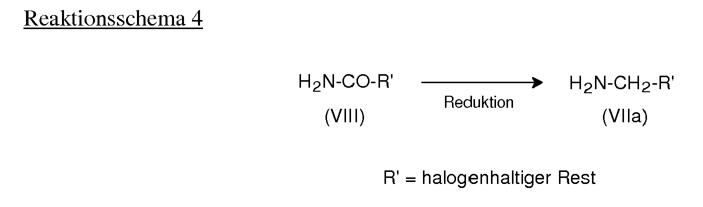

Als Reduktionsmittel eignet sich beispielsweise der bekannte Boran-Dimethylsulfid Komplex (vgl. auch die Herstellung von 2-Chlor-2-fluorethan-1-amin aus kommerziell erhältlichem 2-Chlor-2-fluoracetamid).

Alternativ und in bestimmten Fällen ist auch eine Herstellung von Verbindungen der Formel (V) aus den entsprechenden Aldehyden (A-CHO) und Verbindungen der Formel (VII) mittels reduktiver Aminierung möglich (vgl. Houben-Weyl, Methoden der Organischen Chemie, Bd. XI/1, Georg Thieme Verlag Stuttgart, S. 602). Die Aldehyde (A-CHO) sind z. T. kommerziell erhältlich (vgl. z. B. 6-Chlor-nicotinaldehyd, 6-Fluor-nicotinaldehyd, 6-Brom-nicotinaldehyd, 2-Chlor-1,3-thiazol-5-carbaldehyd) oder können nach literaturbekannten Methoden erhalten werden (vgl. z. B. 6-Methyl-nicotinaldehyd: EP-A2-104876; 2-Chlor-pyrazin-5-caboxaldehyd: DE 3314196 A1).

Der Reaktionsschritt (a) des erfindungsgemäßen Herstellungsverfahrens wird vorzugsweise in Gegenwart eines basischen Reaktionshilfsmittels und in Gegenwart von Verdünnungsmitteln durchgeführt. Verdünnungsmittel werden in einer solchen Menge eingesetzt, dass das Reaktionsgemisch während des ganzen Verfahrens gut rührbar bleibt. Geeignete Verdünnungsmittel sind alle inerten organischen Lösungsmittel (z.B. Halogenkohlenwasserstoffe, insbesondere Chlorkohlenwasserstoffe, wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol; Alkohole wie Methanol, Ethanol, Isopropanol, Butanol); Ether (z.B. Ethylpropylether, Methyl-tert-butylether, *n*-Butylether, Anisol, Phenetol, Cyclohexylmethylether, Dimethylether, Diethylether, Diproplether, Diisopropylether, Di-n-butylether, Diisobutylether, Diisoamylether, Ethylenglycoldimethylether, Tetrahydrofuran, Dioxan, Dichlordiethylether und Polyether des Ethylenoxids und/oder Propylenoxids); Amine (z.B. Trimethyl-, Triethyl-, Tripropyl-, Tributylamin, *N*-Methylmorpholin, Pyridin und Tetramethylendiamin); Nitrokohlenwasserstoffe (z.B. Nitromethan, Nitroethan, Nitropropan, Nitrobenzol, Chlornitrobenzol, o-Nitrotoluol); Nitrile (z.B. Acetonitril, Propionitril, Butyronitril, Isobutyronitril, Benzonitril, m-Chlorbenzonitril) sowie Verbindungen wie Tetrahydrothiophendioxid und Dimethylsulfoxid, Tetramethylensulfoxid, Dipropylsulfoxid, Benzylmethylsulfoxid, Diisobutylsulfoxid, Dibutylsulfoxid, Diisoamylsulfoxid; Sulfone wie Dimethyl-, Diethyl-, Dipropyl-, Dibutyl-, Diphenyl-, Dihexyl-, Methylethyl-, Ethylpropyl-, Ethylisobutyl- und Pentamethylensulfon; aliphatische, cycloaliphatische oder aromatische Kohlenwasserstoffe wie Pentan, Hexan, Heptan, Oktan, Nonan und technische Kohlenwasserstoffe; beispielsweise sogenannte White Spirits mit Komponenten mit Siedepunkten im Bereich von etwa 40°C bis etwa 250°C, Cymol, Benzinfraktionen innerhalb eines Siedeintervalles von etwa 70°C bis etwa 190°C, Cyclohexan, Methylcyclohexan, Petrolether, Ligroin, Octan, Benzol, Toluol, Chlorbenzol, Brombenzol, Nitrobenzol, Xylol; Ester (z.B. Methyl-, Ethyl-, Butyl-, Isobutylacetat, sowie Dimethyl-, Dibutyl-, Ethylencarbonat); Amide (z.B. Hexamethylenphosphorsäuretriamid, Formamid, *N*-Methyl-formamid, *N,N*-Dimethyl-formamid, *N,N*-Dipropyl-formamid, *N,N*-Dibutyl-formamid, *N*-Methyl-pyrrolidon, *N*-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolidindion, *N*-Formyl-piperidin, *N,N'*-1,4-Diformyl-piperazin); Ketone (z.B. Aceton, Acetophenon, Methylethylketon, Methylbutylketon). Es können auch Mischungen der vorgenannten Verdünnungsmittel eingesetzt werden.

In Reaktionsschritt (a) werden als Verdünnungsmittel bevorzugt Ether, insbesondere Methyl-tert-butylether und Dipropylether, Tetrahydrofuran und Dioxan eingesetzt.

Der Reaktionsschritt (a) wird vorzugsweise in Gegenwart eines basische Reaktionshilfsmittel (d.h. Hilfsmittel) eingesetzt. Geeignet sind insbesondere Säurebindemittel wie Amine, insbesondere tertiäre Amine sowie Alkali- und Erdalkaliverbindungen. Beispielhaft seien erwähnt die Hydroxide, Hydride, Oxide, Amide und Carbonate des Lithiums, Natriums, Kaliums, Magnesiums, Calciums und Bariums, ferner weitere basische Verbindungen wie Amidinbasen oder Guanidinbasen wie 7-Methyl-1,5,7-triaza-bicyclo[4.4.0]dec-5-en (MTBD); Diazabicyclo[4.3.0]nonen (DBN), Diazabicyclo[2.2.2]octan (DABCO), 1,8-Diazabicyclo[5.4.0]undecen (DBU), Cyclohexyltetrabutyl-guanidin (CyTBG), Cyclohexyltetramethylguanidin (CyTMG), *N,N,N,N*-Tetramethyl-1,8-naphthalindiamin, Pentamethylpiperidin, tertiäre Amine wie Triethylamin, Trimethylamin, Tribenzylamin, Triisopropylamin, Tributylamin, Tricyclohexylamin, Triamylamin, Trihexylamin, *N,N*-Dimethylanilin, *N,N*-Dimethyl-toluidin, *N,N*-Dimethyl-*p-*aminopyridin, *N*-Methyl-pyrrolidin, *N*-Methyl-piperidin, *N*-Methyl-imidazol, *N*-Methyl-pyrazol, *N-*Methyl-morpholin, *N*-Methyl-hexamethylendiamin, Pyridin, 4-Pyrrolidinopyridin, 4-Dimethylamino-pyridin, Chinolin, α-Picolin, β-Picolin, Isochinolin, Pyrimidin, Acridin, *N,N,N',N'*-Tetramethylendiamin, *N,N,N',N'*-Tetraethylendiamin, Chinoxalin, *N*-Propyl-diisopropylamin, *N*-Ethyldiisopropylamin, *N,N'*-Dimethyl-cyclohexylamin, 2,6-Lutidin, 2,4-Lutidin oder Triethyldiamin. Bevorzugte basische Hilfsmittel sind Lithium-*bis*-(trimethylsilyl)-amid oder Kalium-bis-(trimethylsilyl)-amid.

Zur Durchführung des Reaktionsschrittes (a) setzt man pro Mol Verbindung der allgemeinen Formel (II) im Allgemeinen 0,5 bis 4,0 Mol, vorzugsweise 0,5 bis 4,0 Mol, besonders bevorzugt 0,5 bis 1,0 Mol an Verbindung der allgemeinen Formel (III) ein.

Nach Umsetzung wird der gesamte Reaktionsansatz des Reaktionsschrittes (a) zwischen Wasser und einem geeigneten Halogenkohlenwasserstoff, beispielsweise Dichlormethan, verteilt und mit verdünnter Säure und Base gewaschen. Die nach Aufarbeitung anfallenden Produkte lassen sich durch Umkristallisieren, Vakuumdestillation oder Säulenchromatographie reinigen (vgl. auch die Herstellungsbeispiele).

Der Reaktionsschritt (b) wird vorteilhafterweise in Gegenwart von Verdünnungsmitteln durchgeführt, wobei die oben angeführten Verdünnungsmittel verwendet werden können. Bevorzugt werden Ether wie Tetrahydrofuran oder Dioxan eingesetzt.

Im Reaktionsschritt (b) werden pro Mol Verbindung der allgemeinen Formel (IV) im Allgemeinen 0,5 bis 5,0 Mol, bevorzugt 0,5 bis 3,0 Mol, besonders bevorzugt 0,5 bis 1,5 Mol und ganz besonders bevorzugt ein geringer Überschuss an Aminoverbindung der allgemeinen Formel (V) eingesetzt.

Verdünnungsmittel werden vorteilhaft in einer solchen Menge eingesetzt, dass das Reaktionsgemisch während des ganzen Verfahrens gut rührbar bleibt.

Die Reaktionsdauer des Verfahrens bzw. des Reaktionsschritts (a) oder (b) beträgt im Allgemeinen von 10 Minuten bis 48 Stunden. Die Umsetzungen erfolgen bei Temperaturen zwischen -100°C und +100°C, bevorzugt zwischen -80°C und +80°C, besonders bei -75 °C bis Raumtemperatur.

Es kann grundsätzlich unter Normaldruck gearbeitet werden. Vorzugsweise arbeitet man bei Normaldruck oder bei Drucken bis zu 15 bar und gegebenenfalls unter Schutzgasatmosphäre (Stickstoff, Helium oder Argon).

Nach vollendeter Umsetzung wird der gesamte Reaktionsansatz eingeengt. Die nach Aufarbeitung anfallenden Produkte lassen sich in üblicher Weise durch Umkristallisieren, Vakuumdestillation oder Säulenchromatographie reinigen (vgl. auch die Herstellungsbeispiele).

Selbstverständlich können die geometrischen (beispielsweise die E- oder Z- Isomere) und/oder optisch aktiven Isomere oder entsprechende Isomerengemische der Formel (I) nach einem geeigneten Trennverfahren aufgetrennt werden, beispielsweise mittels Säulenchromatographie ggf. an einer chiralen Phase oder mittels Extraktionsverfahren (z. B. Craig-Verteilung).

Zur Darstellung der erfindungsgemäßen Verbindungen in denen R² für Halogen steht, können alternativ auch Verbindungen der Formel (I) in der R² für Wasserstoff steht, mit Halogenierungsmitteln in Gegenwart von basischen Hilfsmitteln gemäß dem Reaktionsschema 5 umgesetzt werden.

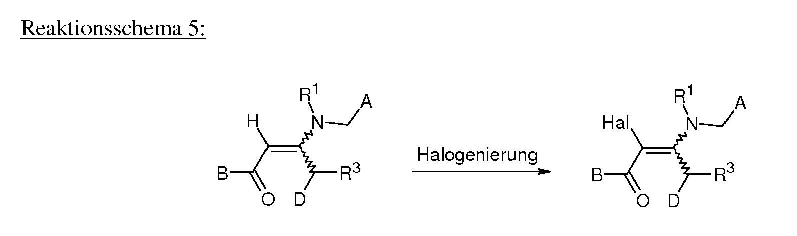

In den als Ausgangsstoffe benötigten Verbindungen der Formel (I) haben A, B, D, R¹ und R³ die weiter oben genannte Bedeutung, der Substituent R² steht für Wasserstoff.

Die Halogenierung wird vorteilhafterweise in Gegenwart von Verdünnungsmitteln durchgeführt.

Die Verdünnungsmittel werden in einer solchen Menge eingesetzt, dass das Reaktionsgemisch während des ganzen Verfahrens gut rührbar bleibt. Geeignete Verdünnungsmittel sind alle unter den Reaktionsbedingungen inerten organischen Lösungsmittel. Bevorzugte Verdünnungsmittel Nitrile wie Acetonitril, Propionitril, Butyronitril, Isobutyronitril, Benzonitril, m-Chlorbenzonitril, besonders bevorzugte Verdünnungsmittel sind Acetonitril, Propionitril oder Butyronitril.

Geeignete Halogenierungsmittel sind dem Fachmann bekannt. Vorzugsweise werden *N*-HalogenVerbindungen (z.B. *N*-Halogenamine wie 1-Chlormethyl-4-fluordiazoniabicyclo[2.2.2]octan-bis-(tetrafluoroborat) (Selectfluor^{®}), *N,N*-Dihalogenamine, *N*-Halogen-carbonsäureamide, *N*-Halogencarbamidsäureester, *N*-Halogen-harnstoff, *N*-Halogen-sulfonylamide, *N*-Halogen-disulfonylamide, *N*-Halogen-sulfonylimide wie *N*-Fluor-bis[(trifluormethyl)sulfonyl]imid und *N*-Halogendicarbonsäureimide wie *N*-Chlor-phthalimid, *N*-Brom-phthalimid, *N*-Iod-phthalimid, *N*-Chlorsuccinimid (NCS), *N*-Brom-succinimid (NBS), *N*-Brom-saccharin oder *N*-Iod-succinimid) eingesetzt. Besonders bevorzugt sind *N*-Halogen-carbonsäurediamide oder 1-Chlormethyl-4-fluordiazoniabicyclo[2.2.2]octan-bis-(tetrafluoroborat) (Selectfluor^{®}).

Die Reaktionsdauer bei diesem Verfahren beträgt im Allgemeinen 10 Minuten bis 48 Stunden.

Die Umsetzung erfolgt bei Temperaturen zwischen -10°C und +100°C, bevorzugt zwischen 0°C und 60°C, besonders bevorzugt zwischen 10°C und Raumtemperatur.

Nach vollendeter Umsetzung wird der gesamte Reaktionsansatz eingeengt. Die nach Aufarbeitung anfallenden Produkte lassen sich in üblicher Weise durch Umkristallisieren, Vakuumdestillation oder Säulenchromatographie reinigen.

Selbstverständlich können auch hier die halogenierten, geometrischen (beispielsweise die E- oder Z-Isomere) und/oder optisch aktiven Isomere oder entsprechende Isomerengemische der Formel (I) nach einem geeigneten Trennverfahren aufgetrennt werden, beispielsweise mittels Säulenchromatographie ggf. an einer chiralen Phase oder mittels Extraktionsverfahren (z. B. Craig-Verteilung).

Die Herstellung von Salzen der erfindungsgemäßen Verbindungen erfolgt nach Standardverfahren. Repräsentative Säureadditionssalze sind beispielsweise solche, die durch Reaktion mit anorganischen Säuren, wie beispielsweise Schwefelsäure, Salzsäure, Bromwasserstoffsäure, Phosphorsäure oder organischen Carbonsäuren wie Essigsäure, Trifluoressigsäure, Zitronensäure, Bernsteinsäure, Buttersäure, Milchsäure, Ameisensäure, Fumarsäure, Maleinsäure, Malonsäure, Camphersäure, Oxalsäure, Phthalsäure, Propionsäure, Glycolsäure, Glutarsäure, Stearinsäure, Salicylsäure, Sorbinsäure, Weinsäure, Zimtsäure, Valeriansäure, Pikrinsäure, Benzoesäure oder organischen Sulfonsäuren wie Methansulfonsäure und 4-Toluolsulfonsäure gebildet werden. Representativ sind auch Salze von erfindungsgemäßen Verbindungen, die aus organischen Basen, wie beispielsweise Pyridin oder Triethylamin gebildet werden, oder solche, die aus anorganischen Basen, wie beispielsweise Hydriden, Hydroxiden oder Carbonaten des Natriums, Kaliums, Lithiums, Calciums, Magnesiums oder Bariums, gebildet werden, wenn die Verbindungen der allgemeinen Formel (I) ein zu dieser Salzbildung geeignetes Strukturelement aufweist.

Synthesemethoden zur Darstellung heterocyclischer *N*-Oxide und tert-Aminen sind bekannt. Sie können mit Peroxysäuren (z.B. Peressigsäure und *meta*-Chlor-perbenzoesäure (MCPBA), Wasserstoffperoxid, Alkylhydroperoxide (z.B. tert-Butylhydroperoxid), Natriumperborat und Dioxiranen (z.B. Dimethyldioxiran) erhalten werden. Diese Methoden sind beispielsweise von T. L. Gilchrist, in Comprehensive Organic Synthesis, Vol. 7, S. 748-750, 1992, S. V. Ley, (Ed.), Pergamon Press; M. Tisler, B. Stanovnik, in Comprehensive Heterocyclic Chemistry, Vol. 3, S. 18-20, 1984, A. J. Boulton, A. McKillop, (Eds.), Pergamon Press; M. R. Grimmett, B. R. T. Keene in Advances in Heterocyclic Chemistry, Vol. 43, S. 149-163, 1988, A. R. Katritzky, (Ed.), Academic Press; M. Tisler, B. Stanovnik, in Advances in Heterocyclic Chemistry, Vol. 9, S. 285-291, 1968, A. R. Katritzky, A. J. Boulton (Eds.), Academic Press; G. W. H. Cheeseman, E. S. G. Werstiuk in Advances in Heterocyclic Chemistry, Vol. 22, S. 390-392,1978, A. R. Katritzky, A. J. Boulton, (Eds.), Academic Press beschrieben.

Die erfindungsgemäßen Verbindungen eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp..

Aus der Ordnung der Chilopoda z.B. Geophilus spp., Scutigera spp..

Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Dermaptera z.B. Forficula auricularia.

Aus der Ordnung der Diplopoda z.B. Blaniulus guttulatus.

Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.

Aus der Klasse der Gastropoda z.B. Arion spp., Biomphalaria spp., Bulinus spp., Deroceras spp., Galba spp., Lymnaea spp., Oncomelania spp., Succinea spp..

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Reticulitermes spp., Odontotermes spp..

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp..

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp..

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie *N*-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnussschalen, Maiskolben und Tabakstängeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP- POE-Ether, Fett- und/oder POP- POE-Addukte, POE- und/oder POP-Polyol Derivate, POE- und/oder POP-Sorbitan- oder Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und/oder physikalische Stabilität verbessernde Mittel.

Die Formulierungen enthalten im allgemeinen zwischen 0,01 und 98 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

Besonders günstige Mischpartner sind z.B. die folgenden:

### Fungizide:

(1) Inhibitoren der Nukleinsäuresynthese, wie beispielsweise Benalaxyl, Benalaxyl-M, Bupirimat, Clozylacon, Dimethirimol, Ethirimol, Furalaxyl, Hymexazol, Metalaxyl, Metalaxyl-M, Ofurace, Oxadixyl und Oxolinsäure.
(2) Inhibitoren der Mitose und Zellteilung, wie beispielsweise Benomyl, Carbendazim, Chlorfenazol, Diethofencarb, Ethaboxam, Fuberidazol, Pencycuron, Thiabendazol, Thiophanat, Thiophanat-Methyl und Zoxamid.
(3) Inhibitoren der Respiration (Atmungsketten-Inhibitoren), wie beispielsweise Diflumetorim als Inhibitor am Komplex I der Atmungskette; Bixafen, Boscalid, Carboxin, Fenfuram, Flutolanil, Fluopyram, Furametpyr, Furmecyclox, Isopyrazam (Mischung aus dem syn-epimeren Razemat 1RS,4SR,9RS und dem anti-epimeren Razemat 1RS,4SR,9SR), Isopyrazam (syn epimeres Razemat 1RS,4SR,9RS), Isopyrazam (syn-epimeres Enantiomer 1R,4S,9R), Isopyrazam (syn-epimeres Enantiomer 1S,4R,9S), Isopyrazam (anti-epimeres Razemat 1RS,4SR,9SR), Isopyrazam (anti-epimeres Enantiomer 1R,4S,9S), Isopyrazam (anti-epimeres Enantiomer 1S,4R,9R),

Mepronil, Oxycarboxin, Penflufen, Penthiopyrad, Sedaxane, Thifluzamid als Inhibitoren am Komplex II der Atmungskette; Amisulbrom, Azoxystrobin, Cyazofamid, Dimoxystrobin, Enestroburin, Famoxadon, Fenamidon, Fluoxastrobin, Kresoxim-Methyl, Metominostrobin, Orysastrobin, Picoxystrobin, Pyraclostrobin, Pyraoxystrobin, Pyrametostrobin, Pyribencarb, Trifloxystrobin als Inhibitoren am Komplex III der Atmungskette.
(4) Entkoppler, wie beispielsweise Binapacryl, Dinocap, Fluazinam und Meptyldinocap.
(5) Inhibitoren der ATP Produktion, wie beispielsweise Fentin Acetat, Fentin Chlorid, Fentin Hydroxid und Silthiofam.
(6) Inhibitoren der Aminosäure- und Protein-Biosynthese, wie beispielsweise Andoprim, Blasticidin-S, Cyprodinil, Kasugamycin, Kasugamycin Hydrochlorid Hydrat, Mepanipyrim und Pyrimethanil.
(7) Inhibitoren der Signaltransduktion, wie beispielsweise Fenpiclonil, Fludioxonil und Quinoxyfen.
(8) Inhibitoren der Lipid- und Membran-Synthese, wie beispielsweise Biphenyl, Chlozolinat, Edifenphos, Etridiazol, Iodocarb, Iprobenfos, Iprodion, Isoprothiolan, Procymidon, Propamocarb, Propamocarb Hydrochlorid, Pyrazophos, Tolclofos-Methyl und Vinclozolin.
(9) Inhibitoren der Ergosterol-Biosynthese, wie beispielsweise Aldimorph, Azaconazol, Bitertanol, Bromuconazol, Cyproconazol, Diclobutrazol, Difenoconazol, Diniconazol, Diniconazol-M, Dodemorph, Dodemorph Acetat, Epoxiconazol, Etaconazol, Fenarimol, Fenbuconazol, Fenhexamid, Fenpropidin, Fenpropimorph, Fluquinconazol, Flurprimidol, Flusilazol, Flutriafol, Furconazol, Furconazol-Cis, Hexaconazol, Imazalil, Imazalil Sulfat, Imibenconazol, Ipconazol, Metconazol, Myclobutanil, Naftifin, Nuarimol, Oxpoconazol, Paclobutrazol, Pefurazoat, Penconazol, Piperalin, Prochloraz, Propiconazol, Prothioconazol, Pyributicarb, Pyrifenox, Quinconazol, Simeconazol, Spiroxamin, Tebuconazol, Terbinafin, Tetraconazol, Triadimefon, Triadimenol, Tridemorph, Triflumizol, Triforin, Triticonazol, Uniconazol, Viniconazol und Voriconazol.
(10) Inhibitoren der Zellwandsynthese, wie beispielsweise Benthiavalicarb, Dimethomorph, Flumorph, Iprovalicarb, Mandipropamid, Polyoxins, Polyoxorim, Prothiocarb, Validamycin A und Valefenalat.
(11) Inhibitoren der Melanin-Biosynthese, wie beispielsweise Carpropamid, Diclocymet, Fenoxanil, Fthalid, Pyroquilon und Tricyclazol.
(12) Resistenzinduktoren, wie beispielsweise Acibenzolar-S-Methyl, Probenazol und Tiadinil.
(13) Verbindungen mit Multisite-Aktivität, wie beispielsweise Bordeauxmischung, Captafol, Captan, Chlorothalonil, Kupfemaphthenat, Kupferoxid, Kupferoxychlorid, Kupferzubereitungen, wie Kupferhydroxid, Kupfersulfat, Dichlofluanid, Dithianon, Dodine und dessen freie Base, Ferbam, Fluorofolpet, Folpet, Guazatin, Guazatinacetat, Iminoctadin, Iminoctadinalbesilat, Iminoctadintriacetat, Mankupfer, Mancozeb, Maneb, Metiram, Zinkmetiram, Kupfer-Oxin, Propamidin, Propineb, Schwefel und Schwefelzubereitungen wie beispielsweise Calciumpolysulfid, Thiram, Tolylfluanid, Zineb und Ziram.
(14) Weitere Verbindungen, wie beispielsweise 2,3-Dibutyl-6-chlorthieno[2,3-d]pyrimidin-4(3H)-on, (2Z)-3-Amino-2-cyano-3-phenylprop-2-ensäureethylester, N-[2-(1,3-Dimethylbutyl)phenyl]-5-fluor-1,3-dimethyl-1H-pyrazol-4-carboxamid, 3-(Difluormethyl)-1-methyl-N-(3',4',5'-trifluorbiphenyl-2-yl)-1H-pyrazol-4-carboxamid, 3-(Difluormethyl)-N-[4-fluor-2-(1,1,2,3,3,3-hexafluorpropoxy)phenyl]-1-methyl-1H-pyrazol-4-carboxamid, (2E)-2-(2-{[6-(3-Chlor-2-methylphenoxy)-5-fluorpyrimidin-4-yl]oxy}phenyl)-2-(methoxyimino)-N-methylethanamid, (2E)-2-{2-[({[(2E,3E)-4-(2,6-Dichlorphenyl)but-3-en-2-yliden]amino}oxy)methyl]phenyl}-2-(methoxyimino)-N-methylethanamid, 2-Chlor-N-(1,1,3-trimethyl-2,3-dihydro-1H-inden-4-yl)pyridin-3-carboxamid, N-(3-Ethyl-3,5,5-trimethylcyclohexyl)-3-(formylamino)-2-hydroxybenzamid, 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, (2E)-2-(Methoxyimino)-N-methyl-2-(2-{[({(1E)-1-[3-(trifluormethyl)phenyl]ethyliden}amino)oxy]methyl}phenyl)ethanamid, (2E)-2-(Methoxyimino)-N-methyl-2-{2-[(E)-({1-[3-(trifluormethyl)phenyl]ethoxy}imino)methyl]phenyl}ethanamid, (2E)-2-{2-[({[(1E)-1-(3-{[(E)-1-Fluor-2-phenylethenyl]oxy}phenyl)ethyliden]amino}oxy)methyl]phenyl}-2-(methoxyimino)-N-methylethanamid, 1-(4-Chlorphenyl)-2-(1H-1,2,4-triazol-1-yl)cycloheptanol, 1-(2,2-Dimethyl-2,3-dihydro-1H-inden-1-yl)-1H-imidazol-5-carbonsaeuremethylester, N-Ethyl-N-methyl-N'-{2-methyl-5-(trifluormethyl)-4-[3-(trimethylsilyl)propoxy]phenyl}imidoformamid, N'-{5-(Difluormethyl)-2-methyl-4-[3-(trimethylsilyl)propoxy]phenyl}-N-ethyl-N-methylimidoformamid, O-{1-[(4-Methoxyphenoxy)methyl]-2,2-dimethylpropyl} 1H-imidazol-1-carbothioat, N-[2-(4-{[3-(4-Chlorphenyl)prop-2-yn-1-yl]oxy}-3-methoxyphenyl)ethyl]-N²-(methylsulfonyl)valinamid, 5-Chlor-7-(4-methylpiperidin-1-yl)-6-(2,4,6-trifluorphenyl)[1,2,4]triazolo[1,5-a]pyrimidin, 5-Amino-1,3,4-thiadiazol-2-thiol, Propamocarb-Fosetyl, 1-[(4-Methoxyphenoxy)methyl]-2,2-dimethylpropyl 1H-imidazol-1-carboxylat, 1-Methyl-N-[2-(1,1,2,2-tetrafluorethoxy)phenyl]-3-(trifluormethyl)-1H-pyrazol-4-carboxamid, 2,3,5,6-Tetrachlor-4-(methylsulfonyl)pyridin, 2-Butoxy-6-iod-3-propyl-4H-chromen-4-on, 2-Phenylphenol und dessen Salze, 3-(Difluormethyl)-1-methyl-N-[2-(1,1,2,2-tetrafluorethoxy)phenyl]-1H-pyrazol-4-carboxamid, 3,4,5-Trichlorpyridin-2,6-dicarbonitril, 3-[5-(4-Chlorphenyl)-2,3-dimethylisoxazolidin-3-yl]pyridin, 3-Chlor-5-(4-chlorphenyl)-4-(2,6-difluorphenyl)-6-methylpyridazin, 4-(4-Chlorphenyl)-5-(2,6-difluorphenyl)-3,6-dimethylpyridazin, 8-Hydroxychinolin, 8-Hydroxychinolinsulfat, Tebufloquin, 5-Methyl-6-octyl-3,7-dihydro[1,2,4]triazolo[1,5-a]pyrimidin-7-amin, 5-Ethyl-6-octyl-3,7-dihydro[1,2,4]triazolo[1,5-a]pyrimidin-7-amin, Ametoctradin, Benthiazol, Bethoxazin, Capsimycin, Carvon, Chinomethionat, Chloroneb, Cufraneb, Cyflufenamid, Cymoxanil, Cyprosulfamide, Dazomet, Debacarb, Dichlorophen, Diclomezin, Dicloran, Difenzoquat, Difenzoquat Methylsulphat, Diphenylamin, Ecomat, Ferimzon, Flumetover, Fluopicolid, Fluoromid, Flusulfamid, Flutianil, Fosetyl-Aluminium, Fosetyl-Calcium, Fosetyl-Natrium, Hexachlorbenzol, Irumamycin, Isotianil, Methasulfocarb, (2E)-2-{2-[({Cyclopropyl[(4-methoxyphenyl)imino]methyl}thio)methyl]phenyl}-3-methoxyacrylsaeuremethylester, Methylisothiocyanat, Metrafenon, (5-Chlor-2-methoxy-4-methylpyridin-3-yl)(2,3,4-trimethoxy-6-methylphenyl)methanon, Mildiomycin, Tolnifanid, N-(4-Chlorbenzyl)-3-[3-methoxy-4-(prop-2-yn-1-yloxy)phenyl]propanamid, N-[(4-Chlorphenyl)(cyano)methyl]-3-[3-methoxy-4-(prop-2-yn-1-yloxy)phenyl]propanamid, N-[(5-Brom-3-chlorpyridin-2-yl)methyl]-2,4-dichlorpyridin-3-carboxamid, N-[1-(5-Brom-3-chlorpyridin-2-yl)ethyl]-2,4-dichlorpyridin-3-carboxamid, N-[1-(5-Brom-3-chlorpyridin-2-yl)ethyl]-2-fluor-4-iodpyridin-3-carboxamid, N-{(Z)-[(Cyclopropylmethoxy)imino][6-(difluormethoxy)-2,3-difluorphenyl]methyl}-2-phenylacetamid, N-{(E)-[(Cyclopropylmethoxy)-imino][6-(difluormethoxy)-2,3-difluorphenyl]methyl}-2-phenylacetamid, Natamycin, Nickel Dimethyldithiocarbamat, Nitrothal-Isopropyl, Octhilinone, Oxamocarb, Oxyfenthiin, Pentachlorphenol und dessen Salze, Phenazin-1-carbonsäure, Phenothrin, Phosphorsäure und deren Salze, Propamocarb Fosetylat, Propanosin-Natrium, Proquinazid, Pyrrolnitrin, Quintozen, S-Prop-2-en-1-yl 5-amino-2-(1-methylethyl)-4-(2-methylphenyl)-3-oxo-2,3-dihydro-1H-pyrazol-1-carbothioat, Tecloftalam, Tecnazene, Triazoxid, Trichlamid, 5-Chlor-N'-phenyl-N'-prop-2-yn-1-ylthiophen-2-sulfonohydrazid, Zarilamid, N-Methyl-2-(1-{[5-methyl-3-(trifluormethyl)-1H-pyrazol-1-yl]acetyl }piperidin-4-yl)-N-[(1R)-1,2,3,4-tetrahydronaphthalen-1-yl]-1,3-thiazol-4-carboxamid, N-Methyl-2-(1-{[5-methyl-3-(trifluormethyl)-1H-pyrazol-1-yl]acetyl }piperidin-4-yl)-N-(1,2,3,4-tetrahydronaphthalen-1-yl)-1,3-thiazol-4-carboxamid, 3-(Difluormethyl)-N-[4-fluor-2-(1,1,2,3,3,3-hexafluorpropoxy)phenyl]-1-methyl-1H-pyrazol-4-carboxamid und Pentyl-{6-[({[(1-methyl-1H-tetrazol-5-yl)(phenyl)methyliden]amino}oxy)methyl]pyridin-2-yl}carbamat.

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Die in dieser Beschreibung mit ihrem "common name" genannten Wirkstoffe sind beispielsweise aus "The Pesticide Manual" 14th Ed., British Crop Protection Council 2006, und der Webseite http://www.alanwood.net/pesticides bekannt.
(1) Acetylcholinesterase (AChE) Inhibitoren, wie beispielsweise Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb; oder
   Organophosphate, z.B. Acephate, Azamethiphos, Azinphos (-methyl, -ethyl), Cadusafos, Chlorethoxyfos, Chlorfenvinphos, Chlormephos, Chlorpyrifos (-methyl), Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Isofenphos, Isopropyl *O*-(methoxyaminothio-phosphoryl) salicylate, Isoxathion, Malathion, Mecarbam, Methamidophos, Methidathion, Mevinphos, Monocrotophos, Naled, Omethoate, Oxydemeton-methyl, Parathion (-methyl), Phenthoate, Phorate, Phosalone, Phosmet, Phosphamidon, Phoxim, Pirimiphos (-methyl), Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triazamate, Triclorfon und Vamidothion.
(2) GABA-gesteuerte Chlorid-Kanal-Antagonisten, wie beispielsweise Organochlorine, z.B. Chlordane und Endosulfan (alpha-); oder Fiprole (Phenylpyrazole), z.B. Ethiprole, Fipronil, Pyrafluprole und Pyriprole.
(3) Natrium-Kanal-Modulatoren/Spannungsabhängige Natrium-Kanal-Blocker, wie beispielsweise Pyrethroide, z.B. Acrinathrin, Allethrin (d-cis-trans, d-trans), Bifenthrin, Bioallethrin, Bioallethrin-S-cyclopentenyl, Bioresmethrin, Cycloprothrin, Cyfluthrin (beta-), Cyhalothrin (gamma-, lambda-), Cypermethrin (alpha-, beta-, theta-, zeta-), Cyphenothrin [(1*R*)-*trans*-Isomere], Deltamethrin, Dimefluthrin, Empenthrin [(*EZ*)-(1*R*)-Isomere], Esfenvalerate, Etofenprox, Fenpropathrin, Fenvalerate, Flucythrinate, Flumethrin, Fluvalinate (tau-), Halfenprox, Imiprothrin, Metofluthrin, Permethrin, Phenothrin [(1*R*)-trans-Isomer], Prallethrin, Profluthrin, Pyrethrine (pyrethrum), Resmethrin, RU 15525, Silafluofen, Tefluthrin, Tetramethrin [(1*R*)- Isomere], Tralomethrin, Transfluthrin und ZXI 8901; oder DDT; oder Methoxychlor.
(4) Nikotinerge Acetylcholin-Rezeptor-Agonisten, wie beispielsweise Neonikotinoide, z.B. Acetamiprid, Clothianidin, Dinotefuran, Imidacloprid, Nitenpyram, Thiacloprid, Thiamethoxam, AKD-1022, Imidaclothiz; oder Nikotin.
(5) Allosterische Acetylcholin-Rezeptor-Modulatoren (Agonisten), wie beispielsweise Spinosyne, z.B. Spinetoram und Spinosad.
(6) Chlorid-Kanal-Aktivatoren, wie beispielsweise Avermectine/Milbemycine, z.B. Abamectin, Emamectin-benzoate, Lepimectin und Milbemectin.
(7) Juvenilhormon-Analoge, z.B. Hydroprene, Kinoprene, Methoprene; oder Fenoxycarb; Pyriproxyfen.
(8) Wirkstoffe mit unbekannten oder nicht spezifischen Wirkmechanismen, wie beispielsweise

Begasungsmittel, z.B. Methylbromid und andere Alkylhalogenide; oder Chloropicrin; Sulfurylfluorid; Borax; Brechweinstein.
(9) Selektive Fraßhemmer, z.B. Pymetrozine; oder Flonicamid.
(10) Milbenwachstumsinhibitoren, z.B. Clofentezine, Diflovidazin, Hexythiazox, Etoxazole.
(11) Mikrobielle Disruptoren der Insektendarmmembran, wie beispielsweise *Bacillus thuringiensis* subsp. *israelensis, Bacillus sphaericus, Bacillus thuringiensis* subsp. *aizawai, Bacillus thuringiensis* subsp. *kurstaki, Bacillus thuringiensis* subsp. *tenebrionis,* und BT-Pflanzen-Proteine, z.B. Cry1Ab, Cry1Ac, Cry1Fa, Cry2Ab, mCry3A, Cry3Ab, Cry3Bb, Cry34/35Ab1.
(12) Inhibitoren der oxidativen Phosphorylierung, ATP-Disruptoren, wie beispielsweise Diafenthiuron; oder Organozinnverbindungen, z.B. Azocyclotin, Cyhexatin, Fenbutatin oxide; oder Propargite; Tetradifon.
(13) Entkoppler der oxidativen Phoshorylierung durch Unterbrechung des H-Protongradienten, wie beispielsweise Chlorfenapyr und DNOC.
(14) Nikotinerge Acetylcholin-Rezeptor-Antagonisten, wie beispielsweise Bensultap, Cartap (-Hydrochlorid), Thiocylam, und Thiosultap (-Natrium).
(15) Inhibitoren der Chitinbiosynthese, Typ 0, wie beispielsweise Benzoylharnstoffe, z.B. Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungsstörende Wirkstoffe, wie beispielsweise Cyromazine.
(18) Ecdysonagonisten/-disruptoren, wie beispielsweise Diacylhydrazine, z.B. Chromafenozide, Halofenozide, Methoxyfenozide und Tebufenozide.
(19) Oktopaminerge Agonisten, wie beispielsweise Amitraz.
(20) Seite-III-Elektronentransportinhibitoren/Seite-II-Elektronentransportinhibitoren, wie beispielsweise Hydramethylnon; Acequinocyl; Fluacrypyrim; oder Cyflumetofen und Cyenopyrafen.
(21) Elektronentransportinhibitoren, wie beispielsweise Seite-I-Elektronentransportinhibitoren, aus der Gruppe der METI-Akarizide, z.B. Fenazaquin, Fenpyroximate, Pyrimidifen, Pyridaben, Tebufenpyrad, Tolfenpyrad; oder Rotenone (Derris).
(22) Spannungsabhängige Natriumkanal-Blocker, z.B. Indoxacarb; Metaflumizone.
(23) Inhibitoren der Acetyl-CoA-Carboxylase, wie beispielsweise Tetronsäure-Derivate, z.B. Spirodiclofen und Spiromesifen; oder Tetramsäure-Derivate, z.B. Spirotetramat.
(24) Seite-IV-Elektronentransportinhibitoren, wie beispielsweise Phosphine, z.B. Aluminiumphosphid, Kalziumphosphid, Phosphin, Zinkphosphid; oder Cyanid.
(25) Ryanodinrezeptor-Effektoren, wie beispielsweise Diamide, z.B. Chlorantraniliprole (Rynaxypyr), Cyantraniliprole (Cyazypyr) und Flubendiamide.

Weitere Wirkstoffe mit unbekanntem Wirkmechanismus, wie beispielsweise Azadirachtin, Amidoflumet, Benzoximate, Bifenazate, Chinomethionat, Cryolite, Dicofol, Flufenerim, Pyridalyl und Pyrifluquinazon; oder folgende bekannte wirksame Verbindungen
4-{[(6-Brompyrid-3-yl)methyl](2-fluorethyl)amino}furan-2(5H)-on (bekannt aus WO 2007/115644), 4-{[(6-Fluorpyrid-3-yl)methyl](2,2-difluorethyl)amino}furan-2(5H)-on (bekannt aus WO 2007/115644), 4-{[(2-Chlor-1,3-thiazol-5-yl)methyl](2-fluorethyl)amino}furan-2(5H)-on (bekannt aus WO 2007/115644), 4-{[(6-Chlorpyrid-3-yl)methyl](2-fluorethyl)amino}furan-2(5H)-on (bekannt aus WO 2007/ 115644), 4-{[(6-Chlorpyrid-3-yl)methyl](2,2-difluorethyl)amino}furan-2(5H)-on (bekannt aus WO 2007/115644), 4-{[(6-Chlor-5-fluorpyrid-3-yl)methyl](methyl)amino}furan-2(5H)-on (bekannt aus WO 2007/115643), 4-{[(5,6-Dichlorpyrid-3-yl)methyl](2-fluorethyl)amino}furan-2(5H)-on (bekannt aus WO 2007/115646), 4-{[(6-Chlor-5-fluorpyrid-3-yl)methyl](cyclopropyl)amino}furan-2(5H)-on (bekannt aus WO 2007/115643), 4-{[(6-Chlorpyrid-3-yl)methyl](cyclopropyl)amino}furan-2(5H)-on (bekannt aus EP-A-0 539 588), 4-{[(6-Chlorpyrid-3-yl)methyl](methyl)amino}furan-2(5H)-on (bekannt aus EP-A-0 539 588), [(6-Chlorpyridin-3-yl)methyl](methyl)oxido-λ⁴-sulfanylidencyanamid (bekannt aus WO 2007/149134), [1-(6-Chlorpyridin-3-yl)ethyl](methyl)oxido-λ⁴-sulfanylidencyanamid (bekannt aus WO 2007/149134) und seine Diastereomere (A) und (B) 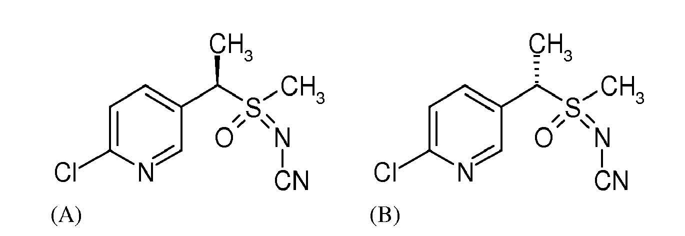 (ebenfalls bekannt aus WO 2007/149134), [(6-Trifluormethylpyridin-3-yl)methyl](methyl)oxido-λ⁴-sulfanylidencyanamid (bekannt aus WO 2007/095229), Sulfoxaflor
(ebenfalls bekannt aus WO 2007/149134), 11-(4-Chlor-2,6-dimethylphenyl)-12-hydroxy-1,4-dioxa-9-azadispiro[4.2.4.2]tetradec-11-en-10-on (bekannt aus WO 2006/089633), 3-(4'-Fluor-2,4-dimethylbiphenyl-3-yl)-4-hydroxy-8-oxa-1-azaspiro[4.5]dec-3-en-2-on (bekannt aus WO 2008/067911) und 1-{2,4-Dimethyl-5-[(2,2,2-trifluoroethyl)sulfinyl]phenyl}-3-(trifluoromethyl)-1H-1,2,4-triazol (bekannt aus WO 1999/55668).

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muss.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepassten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Die erfindungsgemäße Behandlung der Pflanzen und Pflanzenteile mit den Wirkstoffkombinationen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Saatgut, weiterhin durch ein- oder mehrschichtiges Umhüllen.

Insbesondere eignen sich die erfindungsgemäßen Mischungen zur Behandlung von Saatgut. Bevorzugt sind dabei die vorstehend als bevorzugt oder besonders bevorzugt genannten erfindungsgemäßen Kombinationen zu nennen. So entsteht ein großer Teil des durch Schädlinge verursachten Schadens an Kulturpflanzen bereits durch den Befall des Saatguts während der Lagerung und nach dem Einbringen des Saatguts in den Boden sowie während und unmittelbar nach der Keimung der Pflanzen. Diese Phase ist besonders kritisch, da die Wurzeln und Sprosse der wachsenden Pflanze besonders empfindlich sind und bereits ein geringer Schaden zum Absterben der ganzen Pflanze führen kann. Es besteht daher ein insbesondere großes Interesse daran, das Saatgut und die keimende Pflanze durch den Einsatz geeigneter Mittel zu schützen.

Die Bekämpfung von Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen. Es ist weiterhin erstrebenswert, die Menge des eingesetzten Wirkstoffs dahingehend zu optimieren, dass das Saatgut und die keimende Pflanze vor dem Befall durch Schädlinge bestmöglich geschützt wird, ohne jedoch die Pflanze selbst durch den eingesetzten Wirkstoff zu schädigen. Insbesondere sollten Verfahren zur Behandlung von Saatgut auch die intrinsischen insektiziden Eigenschaften transgener Pflanzen einbeziehen, um einen optimalen Schutz des Saatguts und auch der keimenden Pflanze bei einem minimalen Aufwand an Pflanzenschutzmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einem erfindungsgemäßen Mittel behandelt wird. Die Erfindung bezieht sich ebenfalls auf die Verwendung der erfindungsgemäßen Mittel zur Behandlung von Saatgut zum Schutz des Saatguts und der daraus entstehenden Pflanze vor Schädlingen. Weiterhin bezieht sich die Erfindung auf Saatgut, welches zum Schutz vor Schädlingen mit einem erfindungsgemäßen Mittel behandelt wurde.

Einer der Vorteile der vorliegenden Erfindung ist es, dass aufgrund der besonderen systemischen Eigenschaften der erfindungsgemäßen Mittel die Behandlung des Saatguts mit diesen Mitteln nicht nur das Saatgut selbst, sondern auch die daraus hervorgehenden Pflanzen nach dem Auflaufen vor Schädlingen schützt. Auf diese Weise kann die unmittelbare Behandlung der Kultur zum Zeitpunkt der Aussaat oder kurz danach entfallen.

Ein weiterer Vorteil besteht in der synergistischen Erhöhung der insektiziden Wirksamkeit der erfindungsgemäßen Mittel gegenüber dem insektiziden Einzelwirkstoff, die über die zu erwartende Wirksamkeit der beiden einzeln angewendeten Wirkstoffe hinausgeht. Vorteilhaft ist auch die synergistische Erhöhung der fungiziden Wirksamkeit der erfindungsgemäßen Mittel gegenüber dem fungiziden Einzelwirkstoff, die über die zu erwartende Wirksamkeit des einzeln angewendeten Wirkstoffs hinausgeht. Damit wird eine Optimierung der Menge der eingesetzten Wirkstoffe ermöglicht.

Ebenso ist es als vorteilhaft anzusehen, dass die erfindungsgemäßen Mischungen insbesondere auch bei transgenem Saatgut eingesetzt werden können, wobei die aus diesem Saatgut hervorgehenden Pflanzen zur Expression eines gegen Schädlinge gerichteten Proteins befähigt sind. Durch die Behandlung solchen Saatguts mit den erfindungsgemäßen Mitteln können bestimmte Schädlinge bereits durch die Expression des z.B. insektiziden Proteins kontrolliert werden, und zusätzlich durch die erfindungsgemäßen Mittel vor Schäden bewahrt werden.

Die erfindungsgemäßen Mittel eignen sich zum Schutz von Saatgut jeglicher Pflanzensorte wie bereits vorstehend genannt, die in der Landwirtschaft, im Gewächshaus, in Forsten oder im Gartenbau eingesetzt wird. Insbesondere handelt es sich dabei um Saatgut von Mais, Erdnuss, Canola, Raps, Mohn, Soja, Baumwolle, Rübe (z.B. Zuckerrübe und Futterrübe), Reis, Hirse, Weizen, Gerste, Hafer, Roggen, Sonnenblume, Tabak, Kartoffeln oder Gemüse (z.B. Tomaten, Kohlgewächs). Die erfindungsgemäßen Mittel eignen sich ebenfalls zur Behandlung des Saatguts von Obstpflanzen und Gemüse wie vorstehend bereits genannt. Besondere Bedeutung kommt der Behandlung des Saatguts von Mais, Soja, Baumwolle, Weizen und Canola oder Raps zu.

Wie vorstehend bereits erwähnt, kommt auch der Behandlung von transgenem Saatgut mit einem erfindungsgemäßen Mittel eine besondere Bedeutung zu. Dabei handelt es sich um das Saatgut von Pflanzen, die in der Regel zumindest ein heterologes Gen enthalten, das die Expression eines Polypeptids mit insbesondere insektiziden Eigenschaften steuert. Die heterologen Gene in transgenem Saatgut können dabei aus Mikroorganismen wie Bacillus, Rhizobium, Pseudomonas, Serratia, Trichoderma, Clavibacter, Glomus oder Gliocladium stammen. Die vorliegende Erfindung eignet sich besonders für die Behandlung von transgenem Saatgut, das zumindest ein heterologes Gen enthält, das aus Bacillus sp. stammt und dessen Genprodukt Wirksamkeit gegen Maiszünsler und/oder Maiswurzel-Bohrer zeigt. Besonders bevorzugt handelt es sich dabei um ein heterologes Gen, das aus Bacillus thuringiensis stammt.

Im Rahmen der vorliegenden Erfindung wird das erfindungsgemäßes Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD^{®} (z.B. Mais, Baumwolle, Soja), KnockOut^{®} (z.B. Mais), StarLink^{®} (z.B. Mais), Bollgard^{®} (Baumwolle), Nucotn^{®} (Baumwolle) und NewLeaf^{®} (Kartoffel) vertrieben werden. Als Beispiele für Herbizid-tolerante Pflanzen seien Maissorten, Baumwollsorten und Sojasorten genannt, die unter den Handelsbezeichnungen Roundup Ready^{®} (Toleranz gegen Glyphosate z.B. Mais, Baumwolle, Soja), Liberty Link^{®} (Toleranz gegen Phosphinotricin, z.B. Raps), IMI^{®}

(Toleranz gegen Imidazolinone) und STS^{®} (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield^{®} vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitoneal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, dass die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:

Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;

Hautflügler wie Sirex juvencus, Urocerus gigas, Urocerus gigas taignus, Urocerus augur;

Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;

Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:

Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp..

Aus der Ordnung der Chilopoda z.B. Geophilus spp..

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Pemphigus spp., Phylloera vastatrix, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neonicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Herstellungsbeispiele:

### Beispiel 1

### 4-Acetoxy-3-[(6-chlor-pyrid-3-ylmethyl)amino]-but-2-en-carbonsäure-iso-propylester

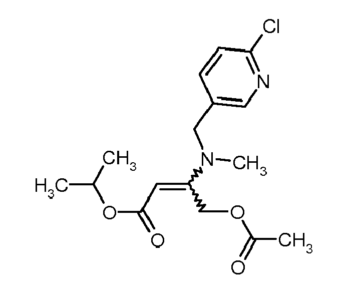

Man legt 124 mg (0.672 mmol) 4-Acetoxybut-2-in-carbonsäure-*iso*-propylester in 5 mL wasserfreiem Tetrahydrofuran (THF) vor und tropft 116 mg (0.739 mmol) 1-(6-Chlorpyridin-3-yl)-*N*-methylmethanamin, gelöst in 1 mL wasserfreiem THF, bei Raumtemperatur zu. Man rührt das Reaktionsgemisch bei Raumtemperatur 16 h und engt im Vakuum ein. Anschließend nimmt man den Rückstand mit Toluol auf und wäscht nacheinander mit verdünnter wässriger Schwefelsäure und Natriumhydrogencarbonat-Lösung. Nach Trocknen über Magnesiumsulfat und Einengen der organischen Phase im Vakuum erhält man 120 mg (50% der Theorie) 4-Acetoxy-3-[(4-chlorbenzyl)(methyl) amino]but-2-en-carbonsäure-*iso*-propylester.

¹H-NMR (CD₃CN, δ, ppm) = 8.21 (m, 1H), 7.56 (m, 1H), 7.36 (m, 1H), 5.45 (s, 2H), 4.91 (sept., 1H), 4.70 (s, 1H), 4.46 (s, 2H), 2.86 (s, 3H), 1.92 (s, 3H), 1.17 (d, 6H).

In der nachstehenden Tabelle 1 sind weitere Verbindungen der allgemeinen Formel (I) aufgeführt.

**Tabelle 1**

| Verbindungen der Formel (Ia), worin A für 6-Chlor-pyrid-3-yl steht | | | | | |
|---|---|---|---|---|---|
| 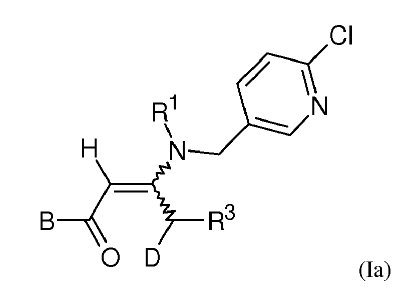 | | | | | |
| **Bsp.-Nr.** | **B** | **R¹** | **R³** | **D** | **Physikalische Daten: ¹H-NMR,** δ **[ppm]^{a}** |
| 2 | OCH₃ | H | CH₃ | O-CO-O-CH₃ (s, | 8.18 (m, 1H), 7.53 (m, 1H), 7.35 (m, 1H), 7.18 (dd, 1H), 4.67 (d, 1H), 4.67 1H), 4.42 (d, 1H), 3.66 (s, 3H), 3.57 (s, 3H), 2.82 (s, 3H), 1.57 (d, 3H) |
| 3 | OCH₃ | H | CH₃ | O-CO-CH₃ | 8.19 (m, 1H), 7.53 (m, 1H), 7.36 (m, 1H), 7.28 (dd, 1H), 4.73 (d, 1H), 4.63 (s, 1H), 4.46 (d, 1H), 3.56 (s, 3H), 2.84 (s, 3H), 1.80 (s, 3H), 1.51 (d, 3H) |
| 4 | OCH₃ | H | H | O-CH₂-C≡CH | 8.21 (m, 1H), 7.57 (m, 1H), 7.34 (m, 1H), 4.96 (s, 2H), 4.70 (s, 1H), 4.53 (s, 2H), 4.20 (d, 2H), 3.56 (s, 3H), 2.86 (s, 3H), 2.65 (t, 1H) |
| 5 | OCH₃ | H | H | O-CH(CH₃)₂ | 8.20 (m, 1H), 7.55 (m, 1H), 7.36 (m, 1H), 5.45 (s, 2H), 4.75 (s, 1H), 4.48 (s, 2H), 3.55 (s, 3H), 2.88 (s, 3H), 2.46 (sept., 1H), 1.07 (d, 6H) |
| 6 | OCH₃ | H | H | O-Cypr | 8.21 (m, 1H), 7.56 (m, 1H), 7.36 (m, 1H), 5.47 (s, 2H), 4.76 (s, 1H), 4.48 (s, 2H), 3.56 (s, 3H), 2.88 (s, 3H), 1.47 (m, 1H), 0.81 (m, 4H) |
| 7 | OC₂H₅ | H | H | O-CO-CH₃ | 8.21 (m, 1H), 7.56 (m, 1H), 7.36 (m, 1H), 5.46 (s, 2H), 4.74 (s, 1H), 4.47 (s, 2H), 4.03 (q, 2H), 2.87 (s, 3H), 2.01 (s, 3H), 1.18 (t, 3H) |
| 8 | OCH(CH₃)₂ | H | H | O-CO-CH₃ | 8.03 (m, 1H), 7.38 (m, 1H), 7.18 (m, 1H), 5.27 (s, 2H), 4.59 (s, 1H), 4.29 (s, 2H), 3.61 (d, 2H), 2.69 (s, 3H), 1.68 (m, 1H), 0.72 (d, 6H) |
| 9 | OCH₃ | H | H | O-CO-C(CH₃)₃ 2H), | 8.20 (m, 1H), 7.55 (m, 1H), 7.35 (m, 1H), 5.43 (s, 2H), 4.75 (s, 1H), 4.49 (s, 3.55 (s, 3H), 2.89 (s, 3H), 1.13 (s, 9H) |
| 10 | OCH₃ | H | H | O-CO-CH₃ | 8.20 (m, 1H), 7.56 (m, 1H), 7.35 (m, 1H), 5.53 (s, 2H), 4.76 (s, 1H), 4.48 (s, 2H), 3.71 (s, 3H), 3.56 (s, 3H), 2.87 (s, 3H) |
| 11 | OCH₃ | C₂H₅ | H | O-CO-CH₃ | 8.21 (m, 1H), 7.57 (m, 1H), 7.35 (m, 1H), 5.43 (s, 2H), 4.71 (s, 1H), 4.46 (s, 2H), 3.53 (s, 3H), 3.32 (q, 2H), 1.95 (s, 3H), 1,13 (t, 3H) |
| 12 | OCH₃ | Cypr | H | O-CO-CH₃ | 8.22 (m, 1H), 7.58 (m, 1H), 7.21 (m, 1H), 5.41 (s, 2H), 5.14 (s, 1H), 4.50 (s, 2H), 3.57 (s, 3H), 2.47 (m, 1H), 2.01 (s, 3H), 0.81 (m, 2H), 0.66 (m, 2H) |
| 13 | OCH₃ | CH₃ | H | O-CO-C₂H₅ | 8.05 (m, 1H), 7.41 (m, 1H), 7.20 (m, 1H), 5.32 (s, 2H), 4.60 (s, 1H), 4.32 (s, 3H), 3.40 (s, 3H), 2.72 (s, 3H), 2.07 (q, 2H), 0.87 (t, 3H) |
| 14 | OCH₃ | CH₃ | H | O-CH₃ | 8.20 (m, 1H), 7.57 (m, 1H), 7.38 (m, 1H), 5.46 (s, 2H), 4.74 (s, 1H), 4.49 (s, 2H), 3.55 (s, 3H), 2.87 (s, 3H), 1.91 (s, 3H) |
| 15 | OCH₃ | CH₃ | H | O-THP | 8.22 (m, 1H), 7.57 (m, 1H), 7.33 (m, 1H), 5.00 (dd, 2H), 4.67 (m, 2H), 4.55 (s, 2H), 3.76 (m, 1H), 3.55 (s, 3H), 3.46 (m, 1H), 2.86 (s, 3H), 1.65-1.35 (m, 6H) |
| 16 | OCH₃ | CH₃ | H | O-CH₂-DMP | 8.14 (m, 1H), 7.49 (m, 1H), 7.25 (m, 1H), 7.11 (m, 1H), 6.46 (m, 1H), 6.43 (M, 1H), 4.92 (s, 2H), 4.67 (s, 1H), 4.49 (s, 2H), 4.47 (S, 2H), 3.77 (s, 3H), 3.72 (s, 3H), 3.55 (s, 3H), 2.82 (s, 3H) |
| 17 | OCH₃ | CH₃ | C₂H₅ | O-CO-OCH₃ | 8.17 (m, 1H), 7.51 (m, 1H), 7.35 (m, 1H), 7.03 (m, 1H), 4.71 (s, 1H), 4.58 (dd, 2H), 3.66 (s, 3H), 3.57 (s, 3H), 2.82 (s, 3H), 1.8m (m, 1H), 1.01 (t, 3H) |
| 18 | OCH₃ | CH₃ | C₂H₅ | O-CO-CH₃ | 8.16 (m, 1H), 7.51 (m, 1H), 7.36 (m, 1H), 7.15 (m, 1H), 4.67 (s, 1H), 4.59 (dd, 2H), 3.56 (s, 3H), 2.83 (s, 3H), 1.82 (m, 4H), 0.98 (t, 3H) |
| 19* | OCH₃ | CH₂CH_{F2} | H | O-CO-CH₃ | 8.21 (m, 1H), 7.57 (dd, 1H), 7.38 (d, 1H), 6.07 (tm, 1H), 5.44 (s, 2H), 4.81 (s, 1H), 4.59 (s, 2H), 3.76 (tm, 2H), 3.53 (s, 3H), 2.01 (s, 3H) |

| | | | | | |
|---|---|---|---|---|---|
| * *(E)*-Isomer; Reinigung mittels präparativer HPLC (RP18-Säule; Laufmittel: Acetonitril/Wassera Gradient); CD₃CN; Cypr = Cyclopropyl, THP = Tetrahydropyran-2-yl, DMP = 2,4-Dimethoxy-phenyl | | | | | |

### Herstellung von Ausgangsverbindungen

### Verbindungen der Formel (IV)

### IV-1

### 4-Acetoxy-but-2-in-carbonsäure-iso-propylester (vgl. auch analoge Synthese R. Tayama, R. Hashimoto Tetrahedron Lett. 48, 7950-7952, 2007)

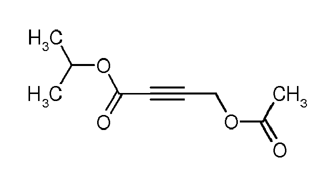

Man löst 2.45 g (25 mmol) Prop-2-in-1-ylacetat in 250 mL wasserfreiem Tetrahydrofuran (THF) und tropft bei -75°C 27.5 mL (27.5 mmol) einer 1 M Lithium-bis-(trimethylsilyl)-amid-Lösung in THF hinzu. Nach 30 min Rühren bei -75°C tropft man langsam bei -75°C 3.68 g (30 mmol) Chlorameisensäure-*iso*-propylester hinzu. Nach 30 min Rühren bei -75°C wärmt man die Reaktionsmischung innerhalb 1 h auf Raumtemperatur auf. Man verteilt zwischen Wasser und Dichlormethan, wäscht die organische Phase nacheinander mit verdünnter wässriger Schwefelsäure und Natriumhydrogencarbonat-Lösung. Nach Trocknen über Magnesiumsulfat und Einengen der organischen Phase im Vakuum wird der Rückstand destilliert. Man erhält 160 mg (3.5% der Theorie) 4-Acetoxy-but-2-in-carbonsäure-*iso*-propylester.

¹H-NMR (CD₃CN, δ, ppm) = 4.88 (sept., 1H), 4.61 (s, 2H), 1.91 (s, 3H), 1.10 (d, 6H).

### IV-2

### 4-Acetoxy-hexin-2-in-carbonsäuremethylester

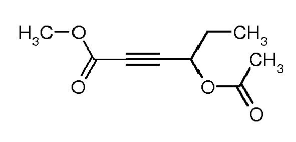

355 mg (2.5 mmol) 4-Hydroxyhex-2-in-carbonsäuremethylester (vgl. J. Kunes et al., Coll. Czech. Chem. Comm. 66, 1809-1830, 2001) und 243 µL (3 mmol) Pyridin werden in 25 mL Dichlormethan gelöst und bei 0°C langsam mit 196 µL (3 mmol) Acetylchlorid versetzt. Nach 16 h bei Raumtemperatur wird nacheinander mit verdünnter wässriger Schwefelsäure und Natriumhydrogencarbonat-Lösung gewaschen. Trocknen über Magnesiumsulfat und Einengen der organischen Phase liefert 410 mg (89% der Theorie) 4-Acetoxyhex-2-in-carbonsäuremethylester.

¹H-NMR (CD₃CN, δ, ppm) = 5.27 (t, 1H), 3.77 (s, 3H), 3.75 (s, 3H), 1.88 (m, 2H), 1.01 (t, 3H).

In der nachstehenden Tabelle 2 sind weitere Verbindungen der allgemeinen Formel (IV) aufgeführt.

**Tabelle 2**

| Verbindungen der Formel | | | | |
|---|---|---|---|---|
| 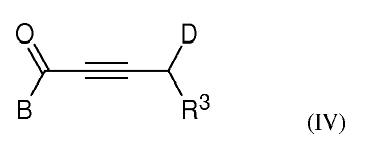 | | | | |
| **Bsp.-Nr.** | **B** | **R³** | **D** | **Physikalische Daten: ¹H-NMR,** δ **[ppm] ^{a} / bekannte Literatur** |
| IV-3 | OCH₃ | H | O-CO-CH(CH₃)₂ | 4.64 (s, 2H), 3.59 (s, 3H), 2.44 (sept., 1H), 0.99 (d, 6H) |
| IV-4 | OCH₃ | H | O-CO-Cypr | 4.64 (s, 2H), 3.58 (s, 3H), 1.48 (m, 1H), 0.78 (m, 4H) |
| IV-5 | OCH₂CH₃ | H | O-CO-CH₃ | 4.78 (s, 2H), 4.22 (s, 2H), 2.06 (s, 3H), 1.27 (t, 3H) |
| IV-6 | OCH₂CH(CH₃)₂ | H | O-CH₃ | 4.62 (s, 2H), 3.79 (d, 2H), 1.88 (s, 3H), 1.76 (m, 1H), 0.78 (d, 6H) |
| IV-7 | OCH3 | H | O-CO-CH₂CH₃ | 4.64 (s, 2H), 3.59 (s, 3H), 2.21 (q, 2H), 0.95 (t, 3H) |
| IV-8 | OCH₃ | H | O-CH₂-DMP | 7.22 (m, 1H), 6.53 (m, 2H), 4.50 (s, 2H), 4.27 (s, 2H), 3.81 (s, 3H), 3.78 (s, 3H), 3.74 (s, 3H) |
| IV-9 | OCH₃ | CH₂CH₃ | O-CH₂-DMP | 5.27 (t, 1H), 3.77 (s, 3H), 3.75 (s, 3H), 1.88 (m, 2H), 1.01 (t, 3H) |

| | | | | |
|---|---|---|---|---|
| ^{a} CD₃CN; Cypr = Cyclopropyl; DMP = 2,4-Dimethoxy-phenyl | | | | |

### Verbindungen der Formel (V)

### V-1

### N-[(6-Chlorpyridin-3-yl)methyl]-2,2-difluorethan-1-amin

41,57 g (256.6 mmol) 2-Chlor-5-chlormethyl-pyridin, 20.80 g (256.6 mmol) 2,2-Difluorethan-1-amin und 35.8 mL (256.6 mmol) Triethylamin werden in 500 mL Acetonitril 21 Stunden bei 45°C gerührt. Nach Einengen der Reaktionsmischung im Vakuum nimmt man mit 1 N wässriger Salzsäure auf und wäscht mit Essigsäureethylester. Die wässrige Phase wird mit 2.5 N wässriger Natriumhydroxid-Lösung alkalisch gestellt und mit Essigsäureethylester mehrmals extrahiert. Einengen der organischen Phase im Vakuum liefert 28.6 g (53 % der Theorie) *N*-[(6-Chlorpyridin-3-yl)methyl]-2,2-difluorethan-1-amin.

¹H-NMR (CD₃CN, δ, ppm) = 2.93 (td, 2 H), 3.80 (s, 2 H), 5.85 (tt, 1 H), 7.33 (d, 1 H), 7.71 (dd, 1H), 8.30 (d, 1 H).

In analoger Weise kann dargestellt werden:

### V-2

### N-[(6-Chlorpyridin-3-yl)methyl]-3-fluorpropan-1-amin

LCMS (*m*/*z*, %) = 203 (MH⁺, 100).

### V-3

### N-[(6-Chlorpyridin-3-yl)methyl]-2-chlor-2-fluorethan-1-amin

LCMS (*m*/*z*, %) = 223 (MH⁺, 100).

### Biologische Beispiele

### Beispiel A: Lucilia cuprina-Test (LUCICU)

### Lösungsmittel: Dimethylsulfoxid

Zur Herstellung einer Wirkstoffzubereitung vermischt man 10 mg Wirkstoff mit 0,5 ml Dimethylsulfoxid und verdünnt das Konzentrat mit Wasser auf die gewünschte Konzentration. Gefäße, die Pferdefleisch enthalten, das mit der Wirkstoffzubereitung der gewünschten Konzentration behandelt wurde, werden mit *Lucilia cuprina* Larven besetzt.

Nach 2 Tagen wird die Abtötung in % bestimmt. Dabei bedeutet 100 %, dass alle Larven abgetötet wurden; 0 % bedeutet, dass keine Larven abgetötet wurden.

Bei diesem Test zeigen die folgenden Verbindungen eine Wirkung von 100 % bei einer Aufwandmenge von 100 ppm:

Bsp Nr : 6, 10, 14

### Beispiel B: Myzus-Test (MYZUPE Spritzbehandlung)

| | | |
|---|---|---|
| Lösungsmittel: | 78 | Gewichtsteile Aceton und 1,5 Gewichtsteile Dimethylformamid |
| Emulgator: | 0,5 | Gewichtsteile Alkylarylpolyglykolether |

Zur Herstellung einer Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach der angegebenen Zeit wird die Wirkung in % bestimmt. Dabei bedeutet 100 %, dass alle Blattläuse abgetötet wurden; 0 % bedeutet, dass keine Blattläuse abgetötet wurden.

Nach 5 Tagen und bei einer Aufwandmenge von 500 g/ha zeigen folgende Verbindungen die angegebene Wirkung:
Verbindung 1 zeigt eine Wirkung von 80 %; Verbindungen 8 und 14 zeigen eine Wirkung von 90 % bei einer Aufwandmenge von 500 g / ha; Verbindungen 2, 3, 5, 6, 7, 9, 10, 11, 12 und 13 zeigen eine Wirkung von 100 %.

Nach 6 Tagen und bei einer Aufwandmenge von 500 g/ha zeigt Verbindung 19 eine Wirkung von 100 %.

### Beispiel C: Meloidogyne incognita-Test (MELGIN)

Lösungsmittel: 78,0 Gewichtsteile Aceton und 1,5Gewichtsteile Dimethylformamid

Emulgator: 0,5 Gewichtsteile Alkylarylpolyglykolether

Zur Herstellung einer Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration. Gefäße werden mit Sand, Wirkstofflösung, *Meloidogyne incognita-*Ei-Larvensuspension und Salatsamen gefüllt. Die Salatsamen keimen und die Pflänzchen entwickeln sich. An den Wurzeln entwickeln sich die Gallen. Nach 14 Tagen wird die nematizide Wirkung an Hand der Gallenbildung in % bestimmt. Dabei bedeutet 100 %, dass keine Gallen gefunden wurden; 0 % bedeutet, dass die Zahl der Gallen an den behandelten Pflanzen der der unbehandelten Kontrolle entspricht.

Bei diesem Test zeigt die Verbindung 10 eine Wirkung von 90 % bei einer Aufwandmenge von 20 ppm.

## Patentansprüche

1. Enaminocarbonylverbindungen der Formel (I) 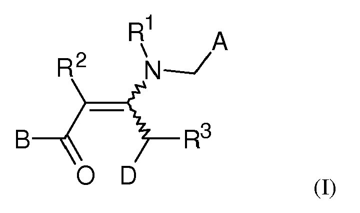 worin
A für einen gegebenenfalls substituierten Heterocyclus steht;
B für einen Rest Z-R⁴ steht, worin
Z für Sauerstoff, Schwefel, oder gegebenenfalls substituierten Stickstoff steht, und
R⁴ für Wasserstoff, C₁-C₆-Alkyl, C₁-C₆-Alkoxy-C₁-C₆-alkyl, C₁-C₆-Alkylthio-C₁-C₆-alkyl, Halogen-C₁-C₆-alkyl, Cyan-C₁-C₆-alkyl, C₂-C₆-Alkenyl, C₂-C₆-Alkinyl, C₃-C₆-Cycloalkyl, C₄-C₁₀-Dicycloalkyl, Tri-C₁-C₆-alkylsilyl, Tri-C₁-C₆-alkylsilyl-C₁-C₆-alkyl, Hetaryl, Hetaryl-C₁-C₆-alkyl, gegebenenfalls substituiertes Aryl, Aryl-C₁-C₆-alkyl oder Aryl-C₁-C₆-alkoxy-C₁-C₆-alkyl steht;
D für Halogen, oder einem Rest T-R⁵ steht, worin
T für Sauerstoff, Schwefel oder gegebenenfalls substituierten Stickstoff steht, und
R⁵ für Wasserstoff, Formyl, C₁-C₆-Alkyl, C₁-C₆-Alkoxy-C₁-C₆-alkyl, Halogen-C₁-C₆-alkoxy-C₁-C₆-alkyl, C₁-C₆-Alkoxy-C₁-C₆-alkoxy-C₁-C₆-alkyl, C₁-C₆-Alkylthio-C₁-C₆-alkyl, C₂-C₆-Alkenyl, C₃-C₆-Cycloalkyl, C₂-C₆- Alkinyl, C₁-C₆-Alkoxycarbonyl, Aryl, Hetaryl, C₁-C₆-Alkylcarbonyl, Aryl-C₁-C₆-alkyl, Hetaryl-C₁-C₆-alkyl, Halogen-C₁-C₆-alkylcarbonyl, Arylcarbonyl, C₁-C₆-Alkylsulfonyl, Halogen-C₁-C₆-alkylsulfonyl, Aryl-C₁-C₆-alkyloxy-C₁-C₆-alkyl, Aryloxy-C₁-C₆-alkyl, Arylsulfonyl oder Tri-C₁-C₆-alkylsilyl steht;
R¹ für Wasserstoff, C₁-C₆-Alkyl, C₂-C₆-Alkenyl, C₁-C₆-Alkinyl, C₃-C₆-Cycloalkyl, C₃-C₆- Cycloalkyl-C₁-C₆-alkyl, Halogen-C₁-C₆-alkyl, Halogen-C₂-C₆-alkenyl, Halogen-C₃-C₆-cycloalkyl, Halogen-C₃-C₆-cycloalkyl-C₁-C₆-alkyl, C₁-C₆-Alkoxy oder Aryl-C₁-C₆-alkyl steht;
R² für Wasserstoff oder Halogen steht; und
R³ für Wasserstoff oder C₁-C₆-Alkyl steht.

2. Verbindungen nach Anspruch 1, wobei
A für einen Heterocyclus der folgenden Formel steht 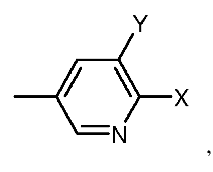 wobei
X für Halogen, C₁-C₆-Alkyl oder Halogen-C₁-C₆-alkyl und
Y für Halogen, C₁-C₆-Alkyl, Halogen-C₁-C₆-alkyl, Halogen-C₁-C₆-alkoxy, Azido oder Cyan stehen;
oder ausgewählt ist unter Pyrid-2-yl; Pyrid-4-yl; oder Pyrid-3-yl, das gegebenenfalls in 6-Position substituiert ist durch Fluor, Chlor, Brom, Iod, Methyl, Difluormethyl, Trifluormethyl oder Trifluormethoxy; Pyridazin-3-yl, das gegebenenfalls in 6-Position substituiert ist durch Chlor oder Methyl; Pyrazin-3-yl; 2-Chlor-pyrazin-5-yl; 1,3-Thiazol-5-yl, welches gegebenenfalls in 2-Position substituiert ist durch Chlor oder Methyl; Tetrahydrofuryl; Pyrimidinyl; Pyrazolyl; Thiophenyl; Oxazolyl; Isoxazolyl; 1,2,4-Oxadiazolyl; Isothiazolyl; 1,2,4-Triazolyl; 1,2,5-Thiadiazolyl, das gegebenenfalls durch Fluor, Chlor, Brom, Cyano, Nitro, substituiert ist, oder mit jeweils gegebenenfalls mit Fluor und/oder Chlor substituiertem C₁-C₄-Alkyl C₁-C₃-Alkylthio, oder C₁-C₃-Alkylsulfonyl substituiert ist;
B für einen Rest Z-R⁴ steht, worin
Z für Sauerstoff oder Schwefel, und
R⁴ für Wasserstoff, C₁-C₆-Alkyl, C₁-C₄-Alkoxy-C₁-C₆-alkyl, C₁-C₄-Alkylthio-C₁-C₆-alkyl, Halogen-C₁-C₆-alkyl, Cyan-C₁-C₄-alkyl, C₂-C₆-Alkenyl, C₂-C₆-Alkinyl, C₃-C₆-Cycloalkyl, Di-C₃-C₆-cycloalkyl-C₁-C₂-alkyl, Tri-C₁-C₆-alkylsilyl, Tri-C₁-C₆-alkylsilyl-C₁-C₂-alkyl, Hetaryl, Hetaryl-C₁-C₂-alkyl, Aryl-C₁-C₂-alkyl, Aryl-C₁-C₂-alkyloxy-C₁-C₃-alkyl, Phenyl oder einem mit Nitro, Halogen, C₁-C₄-Alkoxy, C₁-C₄-Alkylthio, C₁-C₄-Alkylsulfinyl oder C₁-C₄-Alkylsulfonyl substituiertem Phenyl, stehen;
D für Chlor oder Brom oder einem Rest T-R⁵ steht, worin
T für Sauerstoff, und
R⁵ für Wasserstoff, Formyl, C₁-C₆-Alkyl, C₁-C₄-Alkoxy-C₁-C₆-alkyl, Halogen-C₁-C₄-alkoxy-C₁-C₆-alkyl, C₁-C₄-Alkoxy-C₁-C₄-alkoxy-C₁-C₆-alkyl, C₁-C₄-Alkylthio-C₁-C₆-alkyl, C₂-C₆-Alkenyl, C₃-C₆-Cycloalkyl, C₂-C₆-Alkinyl, C₁-C₆-Alkoxycarbonyl, Aryl, Tetrahydropyran-2-yl, 3-Brom-tetrahydropyran-2-yl, Tetrahydrothiopyran-2-yl, 1,4-Dioxan-2-yl, Tetrahydrofuranyl, C₁-C₆-Alkylcarbonyl, Aryl-C₁-C₂-alkyl, Hetaryl-C₁-C₂-alkyl, Halogen-C₁-C₆-alkylcarbonyl, Arylcarbonyl, C₁-C₆-Alkylsulfonyl, Halogen-C₁-C₆-alkylsulfonyl, Aryl-C₁-C₄-alkyloxy-C₁-C₄-alkyl, Aryloxy-C₁-C₄-alkyl, Arylsulfonyl oder Tri-C₁-C₆-alkylsilyl steht;
R¹ für Wasserstoff, C₁-C₆-Alkyl, C₂-C₆-Alkenyl, C₂-C₆-Alkinyl, C₃-C₆-Cycloalkyl, C₃-C₆-Cycloalkyl-C₁-C₄-alkyl, C₁-C₆-Alkoxy, Aryl-C₁-C₄-alkyl oder durch Fluor substituiertes C₁-C₆-Alkyl, C₂-C₆-Alkenyl, C₃-C₆-Cycloalkyl oder C₃-C₆-Cycloalkyl-C₁-C₄-alkyl steht;
R² für Wasserstoff, Fluor oder Chlor steht; und
R³ für Wasserstoff oder C₁-C₆-Alkyl steht.

3. Verbindungen nach Anspruch 1 oder 2, wobei
B für einen Rest Z-R⁴ steht, worin
Z für Sauerstoff, und
R⁴ für Wasserstoff, C₁-C₆-Alkyl, Halogen-C₁-C₆-alkyl, Cyan-C₁-C₄-alkyl, C₃-C₆-Cycloalkyl, Di-C₃-C₆-cycloalkyl-C₁-C₂-alkyl, Tri-C₁-C₆-alkylsilyl, Tri-C₁-C₆-alkylsilyl-C₁-C₂-alkyl, Hetaryl, Phenyl-C₁-C₂-alkyl, Phenyl-C₁-C₂-alkyloxy-C₁-C₃-alkyl, Phenyl oder einem mit Nitro, Halogen, C₁-C₄-Alkoxy, C₁-C₄-Alkylthio, C₁-C₄-Alkylsulfinyl oder C₁-C₄-Alkylsulfonyl substituiertem Phenyl, stehen;
D für Chlor oder Brom oder einem Rest T-R⁵ steht, worin
T für Sauerstoff, und
R⁵ für Wasserstoff, Formyl, C₁-C₆-Alkyl, C₁-C₄-Alkoxy-C₁-C₆-alkyl, Halogen-C₁-C₄-alkoxy-C₁-C₆-alkyl, C₁-C₄-Alkoxy-C₁-C₄-alkoxy-C₁-C₆-alkyl, C₁-C₄-Alkylthio-C₁-C₆-alkyl, C₂-C₆-Alkenyl, C₃-C₆-Cycloalkyl, C₂-C₆-Alkinyl, C₁-C₆-Alkoxycarbonyl, Phenyl, Tetrahydropyran-2-yl, 3-Brom-tetrahydropyran-2-yl, Tetrahydrothiopyran-2-yl, 1,4-Dioxan-2-yl, Tetrahydrofuranyl, C₁-C₆-Alkylcarbonyl, Phenyl-C₁-C₂-alkyl, Hetaryl-C₁-C₂-alkyl, Halogen-C₁-C₆-alkylcarbonyl, Phenylcarbonyl, C₁-C₆-Alkylsulfonyl, Halogen-C₁-C₆-alkylsulfonyl, Phenyl-C₁-C₄-alkyloxy-C₁-C₄-alkyl, Phenyloxy-C₁-C₄-alkyl, Phenylsulfonyl oder Tri-C₁-C₆-alkylsilyl steht;
R¹ für C₁-C₆-Alkyl, C₂-C₆-Alkenyl, C₂-C₆-Alkinyl, C₃-C₆-Cycloalkyl, C₃-C₆-Cycloalkyl-C₁-C₄-alkyl, C₁-C₆-Alkoxy oder durch Fluor substituiertes C₁-C₆-Alkyl, C₂-C₆-Alkenyl, C₃-C₆-Cycloalkyl oder C₃-C₆-Cycloalkyl-C₁-C₄-alkyl steht;
R² für Wasserstoff, Fluor oder Chlor steht; und
R³ für Wasserstoff oder C₁-C₄-Alkyl steht.

4. Verbindungen nach Anspruch 1 oder 2, wobei
B für einen Rest Z-R⁴ steht, worin
Z für Sauerstoff und
R⁴ für Wasserstoff, Methyl, Ethyl, *n*-Propyl, *iso*-Propyl, *n*-Butyl, *iso*-Butyl, *sec-*Butyl, *tert*-Butyl, Methoxyethyl, Methylthiomethyl, 2-Methylthioethyl, 2,2,2-Trichlorethyl, 2-Chlor-ethyl, 2-Brom-ethyl, 2-Iod-ethyl, 2-Cyan-ethyl, Allyl, Methallyl, 3-Buten-1-yl, Propargyl, Cyclopentyl, Cyclohexyl, Dicyclopropylmethyl, Trimethylsilyl, Di-*tert-*butylmethylsilyl, *iso*-Propyldimethylsilyl, Trimethylsilylmethyl, 2-(2'-Pyridyl)ethyl, 4-Picolyl, Benzyl, 4-Methoxy-benzyl, 2,4-Dimethoxy-benzyl, 4-Brom-benzyl, 4-Methylsulfinyl-benzyl, 4-Nitro-benzyl, Benzyloxymethyl, 4-Methylthio-phenyl, 4-Nitro-phenyl oder 2,3,4,5,6-Pentafluor-phenyl stehen;
D für Chlor oder für einen Rest T-R⁵ steht, worin
T für Sauerstoff und
R⁵ für Wasserstoff, Formyl, Methyl, Ethyl, *n*-Propyl, *iso*-Propyl, *n*-Butyl, *iso-*Butyl, *sec*-Butyl, *tert*-Butyl, Allyl, Methoxymethyl, 1-Ethoxy-ethyl, *tert*-Butoxymethyl, Methylthiomethyl, Methoxyethoxymethyl, 1-(2-Chlorethoxy)ethyl, 1-Methyl-1-methoxyethyl, Cyclopropyl, Cyclobutyl, Propargyl, Methoxycarbonyl, Ethoxycarbonyl, Cyclopropyloxycarbonyl, Phenyl, Tetrahydropyran-2-yl, Methylcarbonyl, Ethylcarbonyl, *n*-Propylcarbonyl, *iso*-Propylcarbonyl, *tert*-Butylcarbonyl, Trifluormethylcarbonyl, 4-Nitro-phenylcarbonyl, 2,4-Dinitro-phenyl, 4-Nitrophenyl, Benzyl, 4-Methoxy-benzyl, 2,4-Dimethoxy-benzyl, 3,4-Dimethoxy-benzyl, 4-Nitrobenzyl, 2,6-Dichlorbenzyl, 4-Methoxy-benzyloxymethyl, 4-Nitro-benzyloxymethyl, 2-Picolyl, 4-Picolyl, Methylsulfonyl, Ethylsulfonyl, 4-Methoxy-phenyl, Trifluormethylsulfonyl, *para*-Toluolsulfonyl, Trimethylsilyl, Triethylsilyl, Triisopropylsilyl, Dimethylisopropylsilyl oder *tert-*Butyldimethylsilyl stehen;
R¹ für Methyl, Ethyl, Allyl, Propargyl, 2-Fluor-ethyl, 2,2-Difluor-ethyl, Cyclopropyl, 2-Fluor-cyclopropyl oder Methoxy steht;
R² für Wasserstoff steht; und
R³ für Wasserstoff oder Methyl steht.

5. Verbindungen nach Anspruch 1 oder 2, wobei
B für einen Rest Z-R⁴ steht, worin
Z für Sauerstoff und
R⁴ für Wasserstoff, Methyl, Ethyl, *n*-Propyl, *iso*-Propyl, *n*-Butyl, *iso*-Butyl, *sec-*Butyl, *tert*-Butyl, Methoxyethyl, Methylthiomethyl, 2-Methylthioethyl, 2,2,2-Trichlorethyl, 2-Chlor-ethyl, 2-Brom-ethyl, 2-Iod-ethyl, 2-Cyan-ethyl, Allyl, Methallyl, 3-Buten-1-yl, Propargyl, Cyclopentyl, Cyclohexyl, Dicyclopropylmethyl, Trimethylsilyl, Di-*tert-*butylmethylsilyl, *iso*-Propyldimethylsilyl, Trimethylsilylmethyl, 2-(2'-Pyridyl)ethyl, 4-Picolyl, Benzyl, 4-Methoxy-benzyl, 2,4-Dimethoxy-benzyl, 4-Brom-benzyl, 4-Methylsulfinyl-benzyl, 4-Nitro-benzyl, Benzyloxymethyl, 4-Methylthio-phenyl, 4-Nitro-phenyl oder 2,3,4,5,6-Pentafluor-phenyl stehen;
D für einen Rest T-R⁵ steht, worin
T für Sauerstoff und
R⁵ Methylcarbonyl, Ethylcarbonyl, *tert*-Butylcarbonyl oder Methoxycarbonyl;
R¹ für Methyl, 2,2-Difluor-ethyl und Cyclopropyl steht;
R² für Wasserstoff steht; und
R³ für Wasserstoff steht.

6. Verbindungen nach einem der Ansprüche 1 bis 5, wobei
A für Pyrid-3-yl steht, welches in 6-Position durch Fluor, Chlor, Brom, Methyl, Trifluormethyl oder Trifluormethoxy substituiert ist; oder ausgewählt unter 5,6-Dibrom-pyrid-3-yl, 6-Brom-5-chlor-pyrid-3-yl, 6-Brom-5-fluor-pyrid-3-yl, 5-Chlor-6-iod-pyrid-3-yl, 5,6-Dichlor-pyrid-3-yl, 6-Chlor-5-fluor-pyrid-3-yl, 5-Brom-6-chlor-pyrid-3-yl, 6-Chlor-5-methyl-pyrid-3-yl, 6-Chlor-5-difluormethyl-pyrid-3-yl, 6-Chlor-1,4-pyridazin-3-yl, 2-Chlor-pyrimid-5-yl, 2-Chlor-1,3-thiazol-5-yl, 6-Trifluormethoxypyrid-3-yl, 6-Chlor-1,4-pyridazin-3-yl, 6-Methyl-1,4-pyridazin-3-yl, 2-Chlor-1,3-thiazol-5-yl, 2-Methyl-1,3-thiazol-5-yl, 2-Chlor-pyrazin-5-yl und 2-Chlor-1,3-thiazol-5-yl.

7. Verbindungen nach einem der Ansprüche 1 bis 5, wobei
A für 6-Fluor-pyrid-3-yl, 6-Chlor-pyrid-3-yl, 6-Brom-pyrid-3-yl, 6-Methyl-pyrid-3-yl, 6-Trifluormethyl-pyrid-3-yl, 6-Trifluormethoxypyrid-3-yl, 6-Chlor-1,4-pyridazin-3-yl, 6-Methyl-1,4-pyridazin-3-yl, 2-Chlor-1,3-thiazol-5-yl oder 2-Methyl-1,3-thiazol-5-yl steht.

8. Verbindungen nach einem der Ansprüche 1 bis 5, wobei
A für 6-Fluor-pyrid-3-yl, 6-Chlor-pyrid-3-yl, 6-Brompyrid-3-yl, 6-Chlor-1,4-pyridazin-3-yl oder 2-Chlor-1,3-thiazol-5-yl steht.

9. Verbindungen nach Anspruch 1 , wobei
A für 6-Chlor-pyrid-3-yl, 6-Brom-pyrid-3-yl, oder 6-Chlor-1,4-pyridazin-3-yl steht;
B für OCH₃, OCH₂CH₃, OCH(CH₃)₂ steht;
D für einen Rest T-R⁵ steht, worin
T für Sauerstoff und
R⁵ für Methylcarbonyl, Ethylcarbonyl, *tert*-Butylcarbonyl oder Methoxycarbonyl stehen;
R¹ für Methyl, Cyclopropyl oder 2,2-Difluor-ethyl steht; und
R¹ und R³ jeweils für Wasserstoff stehen.

10. Verfahren zur Herstellung der Verbindungen nach einem der Ansprüche 1 bis 9., umfassend die folgenden Reaktionsschritte
(a) Umsetzung einer Verbindung der Formel (II) 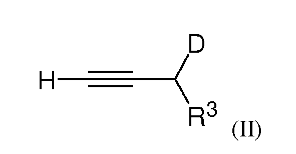 mit einer Verbindung der Formel (III) 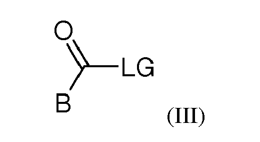 worin
B, D und R³ wie in einem der Ansprüche 1 bis 9 definiert sind; und
LG für Halogen-C₁-C₈-alkoxy, C₁-C₈-Alkanoyloxy, Mercapto, C₁-C₈-Alkylthio, Halogen-C₁-C₈-alkylthio, Halogen steht,
gegebenenfalls in Gegenwart eines Verdünnungsmittels und gegebenenfalls in Gegenwart eines basischen Hilfsmittels zu einer Verbindung der Formel (IV); und
(b) Umsetzen der Verbindungen der Formel (IV) 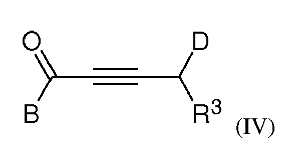 mit einer Verbindungen der Formel (V)
HN(R¹)-CH₂-A (V)
worin die B, D, A und R¹, und R³ wie in einem der Ansprüche 1 bis 9 definiert sind, gegebenenfalls in Gegenwart eines Verdünnungsmittels.

11. Mittel zur Bekämpfung tierischer Schädlinge umfassend mindestens eine Verbindung aus einem der Ansprüche 1 bis 9.

12. Verwendung der Verbindung aus einem der Ansprüche 1 bis 9 zur Bekämpfung tierischer Schädlinge im Agrochemischen Bereich und/oder im Bereich der Tiergesundheit.

13. Verfahren zum Bekämpfen von Schädlingen, **dadurch gekennzeichnet, dass** man eine Verbindung aus einem der Ansprüche 1 bis 9 oder ein Mittel gemäß Anspruch 11 auf die Schädlinge und/oder ihren Lebensraum einwirken lässt.
